# EUROPEAN PATENT APPLICATION

(11) **EP 4 706 634 A1**
(43) Date of publication of application: **11.03.2026**
(21) Application number: 24800110.9
(22) Date of filing: 30.04.2024
(51) Int. Cl.: A61K 8/895, A61K 8/06, A61K 8/31, A61K 8/37, A61Q 1/02, A61Q 1/04, A61Q 1/10, A61Q 1/12, A61Q 5/00, A61Q 17/04, A61Q 19/00

(54) **COSMETIC**

(30) Priority: 02.05.2023 JP 2023076183
(71) Applicant: SHIN-ETSU CHEMICAL CO., LTD., Tokyo 1000005 (JP)
(72) Inventor: AKABANE Emi, Annaka-shi, Gunma 379-0224 (JP); KONISHI Masayuki, Tokyo 100-0005 (JP); KIKUCHI Hiroko, Tokyo 100-0005 (JP)
(74) Representative: Mewburn Ellis LLP
(86) International application number: PCT/JP2024/016663
(87) International publication number: WO 2024/228379

(57) **Abstract**

A cosmetic containing an alkyl-modified silicone and exhibiting an excellent emollient effect, soft focus effect, and feeling upon application, said cosmetic containing a silicone gel obtained by swelling the following (a) component with the following (b) component:
(a) 5-80 mass% of a cross-linked silicone in which a silicone chain having a C8-30 alkyl branch is cross-linked with a silicone chain; and
(b) 20-95 mass% of at least one oil agent selected from (b1) and (b2), where
(b1) is a hydrocarbon oil having a boiling point of 190-260°C and a kinematic viscosity at 25°C of 1.5-10 mm²/s, and
(b2) is an ester oil having an IOB value of 0.05-0.3 and a kinematic viscosity at 25°C of 1.5-25 mm²/s.

## Description

### TECHNICAL FIELD

The present invention relates to a cosmetic containing a silicone gel. In the present invention, a composition for cosmetics may be described as a cosmetic.

### BACKGROUND ART

A silicone gel has been used as a thickener for an oil agent or used to improve usability. In Patent Document 1 and Patent Document 2, non-aqueous and emulsified cosmetics containing an alkyl-modified silicone gel are known.

Patent Document 3 proposes a cosmetic with a roughness correction effect and high usability by combining a silicone gel and an oil-absorbing powder. Patent Document 4 proposes a cosmetic with a soft focus effect and high usability by combining a silicone gel and organosiloxane particles. However, an alkyl-modified silicone gel itself has not been studied, and an optimal base oil also has not been studied.

### PRIOR ART DOCUMENTS

### PATENT DOCUMENTS

Patent Document 1: JP-A 2015-083561
Patent Document 2: WO 2021/100750
Patent Document 3: WO 2018/143061
Patent Document 4: JP-A 2020-075881

### SUMMARY OF INVENTION

### TECHNICAL PROBLEM

The present invention has been made in view of the above circumstances and aims to provide a cosmetic containing an alkyl-modified silicone and having high usability, a good emollient feel, and a good soft focus effect.

### SOLUTION TO PROBLEM

As a result of extensive studies to achieve the above object, the present inventors have completed the present invention by finding that a cosmetic containing a silicone gel in which a specific alkyl-modified silicone is swollen with a specific oil agent has high usability and a good soft focus effect.

Accordingly, the present invention provides the following cosmetics.
1. A cosmetic containing a silicone gel in which the following component (a) is swollen with the following component (b):
   (a) a cross-linked silicone in which a silicone chain having an alkyl branch with 8 to 30 carbon atoms is cross-linked with a silicone chain: 5% to 80% by mass in the gel, and
   (b) one or more oil agents selected from the following (b1) and (b2): 20% to 95% by mass in the gel,
      (b1) a hydrocarbon oil with a boiling point in the range of 190°C to 260°C and a kinematic viscosity in the range of 1.5 to 10 mm²/s at 25°C, and
      (b2) an ester oil with an IOB value in the range of 0.05 to 0.3 and a kinematic viscosity in the range of 1.5 to 25 mm²/s at 25°C.
2. The cosmetic according to 1, wherein the component (a) is one or more selected from a (vinyl dimethicone/lauryl dimethicone) crosspolymer and a (lauryl polydimethylsiloxyethyl dimethicone/bisvinyl dimethicone) crosspolymer.
3. The cosmetic according to 1 or 2, wherein the component (b) is one or more selected from undecane, tridecane, a C13-15 alkane, a cocoalkyl (caprylate/caprate), and (caprylic/capric) triglyceride.
4. The cosmetic according to any one of 1 to 3, wherein a total amount of the component (a) and the component (b) in the silicone gel ranges from 80% to 100% by mass.
5. The cosmetic according to any one of 1 to 4, wherein the silicone gel has a total light transmittance in the range of 80% to 99% at a thickness of 500 µm as measured by a method described in JIS K 7361-1: 1997 and a haze in the range of 40% to 95% as measured by a method described in JIS K 7136: 2000.
6. The cosmetic according to any one of 1 to 5, wherein the cosmetic is a non-aqueous cosmetic.
7. The cosmetic according to any one of 1 to 5, which is a water-in-oil cosmetic, containing:
   the silicone gel: 0.1% to 50% by mass;
   (c) an aqueous component: 30% to 90% by mass; and
   (d) a silicone surfactant other than the component (a): 0.1% to 20% by mass.
8. The cosmetic according to any one of claims 1 to 5, which is an oil-in-water cosmetic, containing:
   the silicone gel: 0.1% to 50% by mass;
   (c) an aqueous component: 40% to 96% by mass; and
   (e) a water-soluble thickener: 0.01% to 10% by mass.

### ADVANTAGEOUS EFFECTS OF INVENTION

The present invention can provide a cosmetic with high usability, a good emollient feel, and a good soft focus effect.

### DESCRIPTION OF EMBODIMENTS

The present invention will be described in detail below. In the present invention, the component name may be described as a cosmetic label name or International Nomenclature of Cosmetic Ingredient (INCI). When the cosmetic label name is equivalent to the INCI, the cosmetic label name or its English description may be omitted.

### [Silicone Gel]

A silicone gel of the present invention is a silicone gel in which the following component (a) is swollen with the following component (b). The component (a) in the present invention is a cross-linked silicone in which a silicone chain having an alkyl branch with 8 to 30 carbon atoms is cross-linked with a silicone chain, and can be used alone or in combination of two or more types thereof. The component (a) is a silicone gel produced by swelling the component (a) with the component (b) described later in advance and is blended in a cosmetic.

The component (a) can be produced by a known method. For example, as in a method described in Japanese Patent No. 4001342, it can be produced by addition polymerization of an organohydrogen polysiloxane having two or more hydroxy groups per molecule and an alkyl branch with 8 to 30 carbon atoms and an organopolysiloxane having two or more vinyl groups per molecule in the presence of a platinum catalyst. The structures of the organohydrogen polysiloxane having an alkyl branch and the organopolysiloxane having vinyl groups to be used may be appropriately adjusted according to the purpose. For example, when it is desired to produce a silicone gel having a high soft focus property, it is preferable to use an organohydrogen polysiloxane having three or more hydroxy groups per molecule and an alkyl branch with 8 to 30 carbon atoms and/or an organopolysiloxane having three or more vinyl groups per molecule. The number of carbon atoms of the alkyl branch preferably ranges from 8 to 16 from the perspective of compatibility with the component (b), more preferably 12. The shape thereof can be appropriately adjusted according to the purpose of use. Specific examples thereof include spherical particles, irregular-shaped particles, resin-like, glutinous, non-fluid gum-like, fluid gum-like, and a viscous liquid, and these forms can be observed visually, with an optical microscope, or with an electron microscope.

The component (a) may be a (vinyl dimethicone/lauryl dimethicone) crosspolymer (INCI: Vinyl Dimethicone/Lauryl Dimethicone Crosspolymer), a (lauryl polydimethylsiloxyethyl dimethicone/bisvinyl dimethicone) crosspolymer (INCI: Lauryl Polydimethylsiloxyethyl Dimethicone/Bis-Vinyl dimethicone Crosspolymer), a polysilicone-22 (INCI: Polysilicone-22) (cetearyl dimethicone/vinyl dimethicone) crosspolymer (INCI: Cetearyl Dimethicone/Vinyl Dimethicone Crosspolymer), a (vinyldimethyl/trimethylsiloxysilicate stearyl dimethicone) crosspolymer (INCI: Vinyldimethyl/Trimethylsiloxysilicate Stearyl Dimethicone Crosspolymer), or the like. Among these, a (vinyl dimethicone/lauryl dimethicone) crosspolymer and a (lauryl polydimethylsiloxyethyl dimethicone/bisvinyl dimethicone) crosspolymer are preferred from the perspective of the compatibility with the component (b), and a (vinyl dimethicone/lauryl dimethicone) crosspolymer is more preferred.

The amount of the component (a) ranges from 5% to 80% by mass, preferably 15% to 60% by mass, more preferably 20% to 50% by mass, in the silicone gel (the entire gel). When the amount of the component (a) is more than 80% by mass, it is difficult to form a gel. When the amount of the component (a) is less than 5% by mass, a sufficient effect cannot be produced when blended in a cosmetic. Furthermore, this range results in good compatibility with the component (b) and also good handling when blended in a cosmetic.

### [Component (b)]

The component (b) of the present invention is one or more oil agents selected from the following (b1) and (b2) and can be used alone or in combination of two or more types thereof. (b1) is a hydrocarbon oil with a boiling point in the range of 190°C to 260°C and a kinematic viscosity in the range of 1.5 to 10 mm²/s at 25°C and can be used alone or in combination of two or more types thereof.

The boiling point in the present invention refers to a boiling point at a pressure of 1,013 hPa. When the hydrocarbon oil is a mixture of two or more substances and the oil agent has a boiling point in a certain range, it is sufficient if the boiling point has the median in this range. The kinematic viscosity in the present invention is a value measured at 25°C using a Cannon-Fenske viscometer described in JIS Z 8803: 2011.

The component (b1) preferably has a boiling point in the range of 190°C to 250°C, more preferably 210°C to 250°C, still more preferably 220°C to 240°C. The kinematic viscosity preferably ranges from 1.5 to 7 mm²/s, more preferably 1.5 to 5 mm²/s, still more preferably 2 to 3.5 mm²/s.

A boiling point of less than 190°C or a kinematic viscosity of less than 1.5 mm²/s at 25°C results in a dry feel and no emollient feel.

A boiling point of more than 260°C or a kinematic viscosity of more than 10 mm²/s at 25°C results in poor usability.

Specific examples of the component (b1) include undecane (INCI) (boiling point = 195°C, kinematic viscosity = 1.6 mm²/s), tridecane (INCI) (boiling point = 235°C, kinematic viscosity = 2.3 mm²/s), a C13-15 alkane (boiling point = 246°C, kinematic viscosity = 2.5 mm²/s), an isoparaffin with 12 to 16 carbon atoms (boiling point = 200°C to 235°C, kinematic viscosity = 1.5 to 3.5 mm²/s), isohexadecane (INCI) (boiling point = 210°C, kinematic viscosity = 4 mm²/s), and a coconut alkane (INCI) (boiling point = 216°C, kinematic viscosity = 1.9 mm²/s). Among these, undecane, tridecane, and a C13-15 alkane are preferred in terms of balance between the emollient feel and usability. The component (b1) may be used alone or in combination of two or more types thereof.

The component (b2) is an ester oil with an IOB value in the range of 0.05 to 0.3 and a kinematic viscosity in the range of 1.5 to 25 mm²/s at 25°C and can be used alone or in combination of two or more types thereof. The IOB value can be calculated as a ratio of an inorganic value to an organic value, that is, "inorganic value/organic value", in an organic conceptual diagram. The organic value and the inorganic value are unique values given to an atom, a functional group, or the like in the organic conceptual diagram. For the details of the organic conceptual diagram, refer to Yoshio Koda, "Yuki Gainenzu -Kiso to Oyo- (Organic Conceptual Diagram -Fundamentals and Applications-)", Sankyo Shuppan Co., Ltd., 1984, pp. 11 to 17.

The IOB value ranges from 0.05 to 0.3, preferably 0.09 to 0.30, more preferably 0.09 to 0.2, still more preferably 0.1 to 0.15, particularly preferably 0.14 to 0.15.

The kinematic viscosity at 25°C ranges from 1.5 to 25 mm²/s, preferably 1.5 to 10 mm²/s, more preferably 1.5 to 7 mm²/s, still more preferably 3 to 7 mm²/s.

An IOB value of more than 0.3 or a kinematic viscosity of more than 25 mm²/s at 25°C may result in no emollient feel or poor usability.

Specific examples of the component (b2) include an alkyl benzoate (C12-15) (IOB = 0.18), isodecyl isononanoate (IOB = 0.16), isononyl isononanoate (IOB = 0.2), cetyl ethylhexanoate (IOB = 0.13), ethylhexyl palmitate (IOB = 0.13), isopropyl myristate (IOB = 0.18), a cocoalkyl (caprylate/caprate) (IOB = 0.15), (caprylic/capric) triglyceride (IOB = 0.3), octyldodecyl myristate (IOB = 0.09), isocetyl myristate (IOB = 0.1), and hexyl laurate (IOB = 0.17). All of these have a kinematic viscosity in the range of 1.5 to 25 mm²/s at 25°C.

As the ester oil, a highly polar ester oil with an IOB value of more than 0.3 and a highly viscous ester oil with a kinematic viscosity of more than 25 mm²/s are also generally used in cosmetics but are not included in the component (b2) in the present invention. Specific examples of such an ester oil include triethylhexanoin (IOB = 0.35), neopentyl glycol diethylhexanoate (IOB = 0.32), and octyldodecyl myristate (kinematic viscosity = 30 mm²/s).

The component (b) can be used alone or in combination of two or more different types thereof. The "two or more different types" may be two or more types of either (b1) or (b2), or (b1) and (b2) may be used in combination. The amount of the component (b) ranges from 20% to 95% by mass, preferably 40% to 85% by mass, more preferably 50% to 80% by mass, in the silicone gel. When the amount of the component (b) is more than 95% by mass, a sufficient effect cannot be produced when blended in a cosmetic. When the amount of the component (b) is less than 20% by mass, it is difficult to form a gel. This range results in good compatibility with the component (a) and also good handling when blended in a cosmetic. From the perspective of ease of production and the stability of the silicone gel, the number of types of the component (b) preferably ranges from 1 to 3, more preferably 1 or 2, still more preferably 1.

### [Other Components in Silicone Gel]

A silicone gel of the present invention is a cross-linked silicone gel containing the component (a) and the component (b). In the silicone gel, the total amount of the component (a) and the component (b) preferably ranges from 80% to 100% by mass, more preferably 99% to 100% by mass, still more preferably 100% by mass in which other components are not substantially blended.

A silicone gel of the present invention may contain a component other than the component (a) and the component (b) without losing the advantages of the present invention. Specific examples thereof include an antioxidant, a pH adjuster, a chelating agent, a surfactant, an oil agent other than the component (b), an oil-soluble gelling agent other than the component (a), and a powder.

A silicone gel of the present invention has a soft focus effect. More specifically, the total light transmittance preferably ranges from 80% to 99% at a thickness of 500 µm as measured by a method described in JIS K 7361-1: 1997, and the haze preferably ranges from 40% to 95% as measured by a method described in JIS K 7136: 2000. The total light transmittance preferably ranges from 80% to 95%, more preferably 85% to 95%, still more preferably 90% to 95%. The haze preferably ranges from 50% to 90%, more preferably 55% to 90%, still more preferably 60% to 90%.

### [Physical Properties of Silicone Gel]

The silicone gel is a gel-like material in which the component (a) is swollen with the component (b). In the present invention, the gel refers to a continuous paste state. The first purpose of forming the gel is to improve the usability by mixing the component (a) with the component (b) having specific physical properties in advance, and the second purpose is to facilitate mixing with another raw material by mixing the component (a) with the component (b) in advance. Thus, fluidity and non-fluidity are not particularly limited.

### [Cosmetic]

A cosmetic of the present invention contains the silicone gel and is applied to various cosmetics. Examples thereof include a cosmetic for external use on the skin, such as a skincare cosmetic, a makeup cosmetic, an antiperspirant cosmetic, or an ultraviolet protection cosmetic, a cosmetic for external use on the hair, such as a hair cosmetic, and a cosmetic for nails. The skincare cosmetic is, for example, a lotion, a milky lotion, a cream, a cleansing, a pack, an oil liquid, a massage cosmetic, a beauty liquid, a beauty oil, a detergent, a deodorant, a hand cream, a lip cream, a wrinkle concealer, or the like. The makeup cosmetic is, for example, a makeup base, a foundation, a concealer, a white powder, a cheek rouge, an eye color, an eye shadow, a mascara, an eyeliner, an eyebrow, a lipstick, or the like. The antiperspirant cosmetic is, for example, an antiperspirant cosmetic of roll-on type, cream type, solution type, stick type, or the like. The ultraviolet protection cosmetic is, for example, a sunscreen oil, a sunscreen emulsion, a sunscreen cream, a preparation produced by imparting a sunscreen effect to the makeup cosmetic, or the like. The hair cosmetic is, for example, a shampoo, a rinse, a hair treatment, a setting agent, a hair dye, a hair fragrance, or the like.

The form of a cosmetic of the present invention may be, for example, any of a powder, a non-aqueous system, a water-in-oil emulsion, an oil-in-water emulsion, a non-aqueous emulsion, a multi-emulsion, such as a W/O/W type or an O/W/O type, and the like. Furthermore, the properties of a cosmetic of the present invention can be selected from various properties, such as liquid, milky liquid, cream, solid, paste, gel, powder, pressed, multilayer, mousse, spray, stick, and pencil.

A method for producing a cosmetic of the present invention only needs to include a step of blending the silicone gel, and a known method for producing each dosage form can be applied. The blending amount of the silicone gel is appropriately selected within the range of 0.1% to 100% by mass of the entire cosmetic.

The cosmetic may be a viscous material or a solid material. The viscous material preferably has a viscosity in the range of 100 to 300,000 mPa·s, 500 to 120,000 mPa·s or less, or 2,000 to 100,000 mPa·s. The viscosity is a value at 25°C measured with a B-type viscometer by a method described in JIS K 7117-1: 1999.

### [Non-Aqueous Cosmetic]

When a silicone gel of the present invention is blended in a non-aqueous cosmetic, the blending amount thereof is not limited, the entire silicone gel of the entire cosmetic can be used as the cosmetic, and the blending amount thereof can range from 0.1% to 95% by mass of the entire cosmetic. When the silicone gel is blended in a non-aqueous cosmetic, the cosmetic can have a high blending amount of the silicone gel and particularly has the advantages of the present invention. Furthermore, oiliness and water resistance can be obtained.

### [Water-In-Oil Cosmetic]

When a silicone gel of the present invention is blended in a water-in-oil cosmetic, the blending amount thereof is preferably, but not limited to, in the range of 0.1% to 50% by mass, more preferably 1% to 35% by mass, still more preferably 3% to 20% by mass, of the entire cosmetic. 0.1% or more by mass can result in further improved usability and a better emollient feel. On the other hand, 50% or less by mass results in improved applicability.

The water-in-oil cosmetic contains, for example, (c) an aqueous component and (d) a silicone surfactant other than the component (a).

### [Component (c)]

The aqueous component may be any aqueous component that can be blended in ordinary cosmetics, and can be used alone or in combination of two or more types thereof. Specific examples thereof include water, an ethanol silicone gel (INCI: Alcohol), isopropanol (label name (INCI: Isopropyl Alcohol), a lower alcohol preferably with 2 to 5 carbon atoms, and a sugar alcohol, such as sorbitol (INCI), maltose (INCI), or xylitol (INCI). Other examples thereof include a polyhydric alcohol, such as BG (label name (INCI: Butylene Glycol)), PG (label name (INCI: Propylene Glycol)), DPG (label name (INCI: Dipropylene Glycol)), pentylene glycol (INCI), 1,10-decanediol (INCI), octanediol (INCI), 1,2-hexanediol (INCI), erythritol (INCI), glycerol (INCI), diglycerol (INCI), or poly(ethylene glycol); and a humectant, such as glucose (INCI), glyceryl glucoside (INCI), betaine (INCI), chondroitin sulfate Na (label name (INCI: Sodium Chondroitin Sulfate)), PCA-Na (label name (INCI: Sodium PCA)), methyl gluceth-10 (INCI), methyl gluceth-20 (INCI), hyaluronic acid, egg-yolk lecithin, soybean lecithin, phosphatidylcholine, phosphatidyl ethanolamine, phosphatidyl serine, phosphatidyl glycerol, phosphatidyl inositol, or sphingophospholipid. In particular, when a polyhydric alcohol is blended, the blending amount thereof preferably ranges from 1% to 50% by mass, more preferably 2% to 30% by mass, still more preferably 3% to 25% by mass, of the entire cosmetic.

When the aqueous component (c) is blended, the blending amount thereof preferably ranges from 30% to 90% by mass, more preferably 50% to 88% by mass, still more preferably 60% to 85% by mass, of the entire cosmetic. 30% or more by mass can result in freshness, and 90% or less by mass results in improved emulsion stability.

### [Component (d)]

The water-in-oil cosmetic preferably contains (d) a silicone surfactant other than the component (a). The HLB of the surfactant is preferably, but not limited to, in the range of 2 to 14.5 for the purpose of maintaining the water resistance of the cosmetic. The component (d) may be a non-cross-linked silicone surfactant or a cross-linked silicone surfactant and can be used alone or in combination of two or more types thereof.

The non-cross-linked silicone surfactant is a silicone surfactant in which part of the methyl groups in a linear or branched silicone main chain is substituted with a hydrophilic group of poly(ethylene glycol), polyglycerol, or the like, and specific examples thereof preferably include a linear or branched polyoxyethylene-modified organopolysiloxane, a linear or branched polyoxyethylene-polyoxypropylene-modified organopolysiloxane, a linear or branched polyoxyethylene-alkyl-co-modified organopolysiloxane, a linear or branched polyoxyethylene-polyoxypropylene-alkyl-co-modified organopolysiloxane, a linear or branched polyglycerol-modified organopolysiloxane, a linear or branched polyglycerol-alkyl-co-modified organopolysiloxane, and a linear or branched pyrrolidone-modified organopolysiloxane. PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 disiloxane dimethicone (INCI), polyglyceryl-3 polydimethylsiloxyethyl dimethicone (INCI), lauryl polyglyceryl-3 polydimethylsiloxyethyl dimethicone (INCI), bisbutyl dimethicone polyglyceryl-3 (INCI), and the like are mentioned.

Examples of commercial products thereof include KF-6011, KF-6011P, KF-6012, KF-6015, KF-6017, KF-6043, KF-6028, KF-6038, KF-6048, KF-6100, KF-6104, KF-6106, KF-6105, and KF-6115 each manufactured by Shin-Etsu Chemical Co., Ltd.

The cross-linked silicone surfactant is, for example, a cross-linked polyether-modified silicone, such as a (dimethicone/(PEG-10/15)) crosspolymer (INCI), a (PEG-15/lauryl dimethicone) crosspolymer (INCI), a (PEG-10/lauryl dimethicone) crosspolymer (INCI), or a (PEG-15/lauryl polydimethylsiloxyethyl dimethicone) crosspolymer (INCI), a cross-linked polyglycerol-modified silicone, such as a (dimethicone/polyglycerol-3) crosspolymer (INCI), a (lauryl dimethicone/polyglycerol-3) crosspolymer (INCI), or a (polyglycerol-3/lauryl polydimethylsiloxyethyl dimethicone) crosspolymer (INCI), or the like.

When a cross-linked silicone surfactant is used, in a composition composed of the cross-linked silicone surfactant and an oil agent that is liquid at room temperature, the cross-linked silicone surfactant preferably contains the liquid oil agent in an amount equal to or greater than its own weight and swells in the liquid oil. As the liquid oil agent, any of the component (b) and (1) a liquid oil in an oil agent as an optional component described later can be suitably used.

Examples of commercially available cross-linked silicone surfactants that contain a liquid oil agent and swell include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z each manufactured by Shin-Etsu Chemical Co., Ltd.

When the component (d) is blended, the blending amount thereof preferably ranges from 0.1% to 20% by mass, more preferably 0.1% to 7.5% by mass, still more preferably 1% to 5% by mass, of the entire cosmetic. 0.1% or more by mass can result in a sufficient dispersion or emulsification function, and 20% or less by mass can result in a cosmetic with a further suppressed sticky feel.

### [Oil-in-Water Cosmetic]

When a silicone gel of the present invention is blended in an oil-in-water cosmetic, the blending amount thereof is preferably, but not limited to, in the range of 0.1% to 50% by mass, more preferably 0.1% to 35% by mass, still more preferably 1% to 25% by mass, of the entire cosmetic. 0.1% or more by mass may result in further improved usability, and more than 50% by mass may result in poor applicability.

The aqueous component (c) is typically blended in an oil-in-water cosmetic, and when blended the blending amount thereof preferably ranges from 40% to 96% by mass, more preferably 50% to 90% by mass, still more preferably 65% to 85% by mass. Less than 30% by mass may result in difficulty in feeling freshness, and more than 96% by mass may result in difficulty in obtaining an emollient feel.

In an oil-in-water cosmetic, a water-soluble thickener (e) is preferably blended in the aqueous component (c). The water-soluble thickener can be used alone or in combination of two or more types thereof. Specific examples of the water-soluble thickener (e) include a plant polymer, such as gum arabic, tragacanth, galactan, carob gum, guar gum, karaya gum, carrageenan, pectin, agar, quince seed (quince), starch (rice, maize, potato, wheat, or the like), algal colloid, gum tragacanth, or locust bean gum, a microbial polymer, such as xanthan gum, dextran, succinoglycan, or pullulan, an animal polymer, such as collagen, casein, albumin, or gelatin, a starch polymer compound, such as carboxymethyl starch or methylhydroxypropyl starch, a cellulose polymer, such as methyl cellulose, ethyl cellulose, methylhydroxypropyl cellulose, carboxymethyl cellulose, hydroxymethylcellulose, hydroxypropyl cellulose, nitrocellulose, sodium cellulose sulfate, sodium carboxymethyl cellulose, crystalline cellulose, cationic cellulose, or a cellulose powder, or an alginate polymer, such as sodium alginate or propylene glycol alginate, a vinyl polymer, such as poly(vinyl methyl ether) or a carboxyvinyl polymer, an acrylic polymer, such as a polyoxyethylene polymer, polyoxyethylene-polyoxypropylene copolymer polymer, sodium polyacrylate, polyethyl acrylate, polyacrylamide, or an acryloyldimethyltaurine salt copolymer, another synthetic water-soluble polymer, such as polyethyleneimine or a cationic polymer, and an inorganic water-soluble polymer, such as bentonite, magnesium aluminum silicate, montmorillonite, beidellite, nontronite, saponite, hectorite, or silicic anhydride.

Among these, one or two or more water-soluble thickeners selected from a plant polymer, a microbial polymer, an animal polymer, a starch polymer, a cellulose polymer, an alginate polymer, a polyoxyethylene-polyoxypropylene copolymer polymer, an acrylic polymer, and an inorganic water-soluble polymer are preferred.

When (d) a silicone surfactant other than the component (a) is blended, the blending amount thereof preferably ranges from 0.01% to 10% by mass, more preferably 0.05% to 5% by mass, still more preferably 0.1% to 2% by mass, of the entire cosmetic. 0.01% or more by mass results in improved stability, and 10% or less by mass results in further improved usability.

A cosmetic of the present invention may contain various components used in ordinary cosmetics without losing the advantages of the present invention. For example, it may contain (1) an oil agent other than the component (b), (2) a surfactant other than the component (d), (3) a powder, (4) a composition composed of a cross-linked organopolysiloxane and an oil agent that is liquid at room temperature other than a silicone gel containing the component (a) and the component (b), (5) a film-forming agent, (6) an ultraviolet absorber or scattering agent, and (7) other additive agents. These may be used alone or in an appropriate combination of two or more thereof. The amount is appropriately selected.

The component (a) and the component (b) of the present invention are blended in the cosmetic in the form of the silicone gel, and the component (a) may or may not be separately contained in the cosmetic in addition to the silicone gel in which the component (a) is swollen in advance with the component (b) of the present invention. When contained, it is preferable to swell by containing the liquid oil agent in an amount equal to or greater than its own weight. As the liquid oil agent, any liquid oil in the oil agent (1) as an optional component can be suitably used.

Examples of a commercially available composition composed of the component (a) and an oil agent that is liquid at room temperature include KSG-41A, KSG-42A, KSG-43, KSG-44, KSG-042Z, KSG-045Z, and KSG-048Z each manufactured by Shin-Etsu Chemical Co., Ltd. The amount of the component (a) in the cosmetic is appropriately selected and is preferably, but not limited to, in the range of 0.01% to 10% by mass, more preferably 0.1% to 5% by mass.

In addition to the silicone gel in which the component (a) is swollen in advance with the component (b) of the present invention, the component (b) may or may not be separately contained in the cosmetic. The amount of the component (b) in the cosmetic is appropriately selected and is preferably, but not limited to, in the range of 0.1% to 50% by mass, more preferably 0.5% to 35% by mass, still more preferably 0.5% to 20% by mass.

The preferred blending amounts of the components (c), (d), and (e) are as described above, and they can be blended in any form of a non-aqueous cosmetic, a water-in-oil cosmetic, an oil-in-water cosmetic, and the like.

### (1) Oil Agent other than Component (b)

The oil agent may be volatile or non-volatile, may be any of a solid, a semi-solid, and a liquid at room temperature (25°C), and is, for example, a silicone oil, a silicone wax, a natural animal or plant oil or fat, a semi-synthetic fat or oil, a hydrocarbon oil, a higher alcohol, a fatty acid, an ester oil, a fluorinated oil agent, an ultraviolet absorber, or the like.

### • Silicone Oil

The silicone oil is, for example, an alkyl-modified silicone, such as dimethicone (INCI), trisiloxane (INCI), methyl trimethicone (INCI), ethyl trisiloxane (INCI), ethyl methicone (INCI), or hexyl dimethicone (INCI), a long-chain alkyl-modified silicone, such as caprylyl methicone (INCI), a low-viscosity to high-viscosity linear or branched organopolysiloxane, such as phenyl trimethicone (INCI), diphenyl dimethicone (INCI), diphenylsiloxyphenyl trimethicone (INCI), tetraphenyldimethyl disiloxane (INCI), or methyl hydrogen polysiloxane, a cyclic organopolysiloxane, such as cyclotetrasiloxane (INCI), cyclopentasiloxane (INCI), or cyclohexasiloxane (INCI), an amino-modified organopolysiloxane, such as amodimethicone (INCI) or aminopropyl dimethicone (INCI), a pyrrolidone-modified organopolysiloxane, such as PCA dimethicone (INCI), a silicone rubber, such as a pyrrolidonecarboxylic acid-modified organopolysiloxane, a gum-like dimethyl polysiloxane with a high degree of polymerization, a gum-like amino-modified organopolysiloxane, or a gum-like dimethylsiloxane-methylphenylsiloxane copolymer, a low-viscosity organopolysiloxane solution of a silicone gum or rubber, an amino acid-modified silicone, a fluorine-modified silicone, a silicone resin, or a dissolved product of a silicone resin.

Examples of commercially available silicone oils include TMF-1.5, KF-96L-1cs, KF-96L-1.5cs, KF-96L-2cs, KF-96A-6cs, KF-4422, KF-4418, KF-54, KF-54HV, KF-56A, and KF-995 each manufactured by Shin-Etsu Chemical Co., Ltd.

### • Solid Oily Component

In the present invention, when it is desired to solidify the cosmetic, an oily component that is solid at 25°C is preferably blended. The oily component that is solid at 25°C preferably has a melting point of 40°C or more, more preferably 60°C to 110°C, may be a wax, a hydrocarbon, an ester, a higher alcohol, or a higher fatty acid, and may be any raw material that can be blended in ordinary cosmetics. Specific examples thereof include a plant wax, such as carnauba wax (INCI: Copernicia Cerifera (Carnauba) Wax), sugarcane wax (INCI: Saccharum Officinarum (Sugarcane) Wax), candelilla wax (INCI: Euphorbia Cerifera (Candelilla) Wax), refined candelilla wax, rice wax, jojoba wax (INCI: Simmondsia Chinensis Seed Wax), kapok wax, rice bran wax (INCI: Oryza Sativa (Rice) Bran Wax), bayberry wax (INCI: Myrica Cerifera (Bayberry) Fruit Wax), shea butter (INCI: Butyrospermum Parkii (Shea) Butter), cocoa butter (INCI: Theobroma Cacao (Cocoa) Seed Butter), Japan wax (INCI: Rhus Succedanea Fruit Wax), montan wax (INCI: Montan Wax), or hydrogenated castor oil isostearate, an animal wax, such as beeswax (INCI: Bees Wax), beef tallow, beef bone fat, lard (INCI: Lard), horse fat (INCI: Horse Fat), mutton tallow, lanolin (INCI: Lanolin), Chinese wax, shellac wax (INCI: Shellac Wax), or spermaceti, a semi-synthetic wax, such as a lanolin ester, a lanolin fatty acid ester, or a beeswax ester, a hydrogenated oil, such as hydrogenated castor oil or hydrogenated coconut oil, a hydrocarbon wax, such as solid paraffin, polyethylene, ceresin, ozokerite, or microcrystalline wax, a wax ester, such as synthetic beeswax, an amino acid stearyl alcohol, such as dioctyldodecyl lauroylglutamate, dioctyldodecyl lauroylglutamate, or dioctyldodecyl lauroylglutamate, a fatty acid, such as stearic acid or behenic acid, a silicone wax, such as an acrylic silicone resin of an acrylic-silicone graft or block copolymer (an acrylic-silicone graft copolymer KP-561P, 562P, or the like each manufactured by Shin-Etsu Chemical Co., Ltd.), and a derivative thereof, and one or two or more selected from these are preferred.

### • Natural Animal or Vegetable Oil Agent and Semi-Synthetic Oil Agent

Examples of a natural animal or vegetable oil agent and a semi-synthetic oil agent include a germ oil, such as avocado oil (label name (INCI: Persea Gratissima (Avocado) Oil)), linseed oil (label name (INCI: Linum Usitatissimum (Linseed) Seed Oil)), almond oil (label name (INCI: Prunus Amygdalus Dulcis (Sweet Almond) Oil)), perilla oil (label name), olive oil (label name (INCI: Olea Europaea (Olive) Fruit Oil)), Torreya californica oil (label name (INCI: Torreya Californica (California Nutmeg) Oil)), citronella oil (label name (INCI: Cymbopogon Nardus (Citronella) Oil)), Khaya seed oil (label name (INCI: Torreya Nucifera Seed Oil)), apricot kernel oil (label name (INCI: Kyounin Yu)), wheat germ oil (label name (INCI: Triticum Vulgare (Wheat) Germ Oil)), sesame oil (label name (INCI: Sesamum Indicum (Sesame) Seed Oil)), rice germ oil (label name (INCI: Oryza Sativa (Rice) Germ Oil)), rice bran oil (label name (INCI: Oryza Sativa (Rice) Bran Oil)), sasanqua oil (label name (INCI: Camellia Kissi Seed Oil)), safflower oil (label name (INCI: Carthamus Tinctorius (Safflower) Seed Oil)), soybean oil (label name (INCI: Glycine Soja(Soybean)Oil)), Camellia sinensis seed oil (label name (INCI: Camellia Sinensis Seed Oil)), camellia oil (label name (INCI: Camellia Japonica Seed Oil)), evening primrose oil (label name (INCI: Oenothera Biennis (Evening Primrose) Oil)), rapeseed oil (label name), or corn germ oil (label name (INCI: Zea Mays (Corn) Germ Oil)), a natural vegetable oil, such as persic oil (label name), palm oil (label name (INCI: Elaeis Guineensis (Palm) Oil)), palm kernel oil (label name (INCI: Elaeis Guineensis (Palm) Kernel Oil)), castor oil (label name (INCI: Ricinus Communis (Castor) Seed Oil)), sunflower seed oil (label name (INCI: Helianthus Annuus (Sunflower) Seed Oil)), grape seed oil (label name (INCI: Vitis Vinifera (Grape) Seed Oil)), jojoba seed oil (label name (INCI: Simmondsia Chinensis (Jojoba) Seed Oil)), macadamia seed oil (label name (INCI: Macadamia Ternifolia Seed Oil)), meadowfoam oil (label name (INCI: Limnanthes Alba (Meadowfoam) Seed Oil)), cotton seed oil (label name (INCI: Gossypium Herbaceum (Cotton) Seed Oil)), coconut oil (label name (INCI: Cocos Nucifera (Coconut) Oil)), or peanut oil (label name (INCI: Arachis Hypogaea (Peanut) Oil)), a natural animal oil, such as shark liver oil (label name (INCI: Shark Liver Oil)), cod liver oil (label name (INCI: Cod Liver Oil)), fish liver oil (label name (INCI: Fish Liver Oil)), turtle oil (label name (INCI: Turtle Oil)), mink oil (label name (INCI: Mink Oil)), or yolk oil (label name (INCI: Egg Oil)), and a semi-synthetic fat or oil, such as hydrogenated coconut oil (label name (INCI: Hydrogenated Coconut Oil)) or liquid lanolin (label name (INCI: Lanolin Oil)).

### • Hydrocarbon Oil other than Component (b1)

A hydrocarbon oil other than the component (b1) is, for example, a linear or branched hydrocarbon oil and may be a volatile hydrocarbon oil or a non-volatile hydrocarbon oil. Specific examples thereof include an olefin oligomer (INCI), an isoparaffin, such as a (C13, 14) isoparaffin (INCI), isododecane (INCI), dodecane (INCI), hydrogenated polyisobutene (label name (INCI: Hydrogenated Polyisobutene)), such as hydrogenated polydecene (label name (INCI: Hydrogenated Polydecene)) liquid isoparaffin or heavy liquid isoparaffin, and an alkane, such as squalane (INCI) or mineral oil (INCI).

### • Higher Alcohol

The higher alcohol is, for example, a linear saturated alcohol with 6 or more carbon atoms, such as lauryl alcohol (INCI), hexyldecanol (INCI), oleyl alcohol (INCI), isostearyl alcohol (INCI), octyldodecanol (INCI), decyltetradecanol (INCI), myristyl alcohol (INCI), cetyl alcohol (INCI), stearyl alcohol (INCI), or behenyl alcohol (INCI), or batyl alcohol (INCI). Also included are sterols, such as cholesterol (INCI), sitosterol (label name (INCI: Beta-Sitosterol)), phytosterol (INCI), lanosterol (INCI), and the like.

### • Ester Oil other than (b2)

The ester oil may be diisobutyl adipate (label name (INCI: Diisobutyl Adipate)), glycol diethylhexanoate (label name (INCI: Glycol Diethylhexanoate)), trimethylolpropane triethylhexanoate (label name (INCI: Trimethylolpropane Triethylhexanoate)), pentaerythrityl tetraethylhexanoate (label name (INCI: Pentaerythrityl Tetraethylhexanoate)), neopentyl glycol diethylhexanoate (label name (INCI: Neopentyl Glycol Diethylhexanoate)), triethyl citrate (label name (INCI: Triethyl Citrate)), diethylhexyl succinate (label name (INCI: Diethylhexyl Succinate)), amyl acetate (label name (INCI: Amyl Acetate)), ethyl acetate (label name (INCI: Ethyl Acetate)), butyl acetate (label name (INCI: Butyl Acetate)), diisopropyl sebacate (label name (INCI: Diisopropyl Sebacate)), cetyl lactate (label name (INCI: Cetyl Lactate)), myristyl lactate (label name (INCI: Myristyl Lactate)), isopropyl lauroyl sarcosinate (label name (INCI: Isopropyl Lauroyl Sarcosinate)), or the like.

Among the ester oils, a glyceride oil may be triethylhexanoin (INCI), (caprylic/capric/succinic) triglyceride (label name (INCI: Caprylic/Capric/Succinic Triglyceride)), (caprylic/capric) glyceride (label name (INCI: Caprylic/Capric Glycerides)), or the like.

### • Fluorinated Oil Agent

The fluorinated oil agent may be perfluorodecalin (INCI), perfluorononyl dimethicone (INCI), perfluoromethylcyclopentane (INCI), or the like.

### • Ultraviolet Absorber

The ultraviolet absorber may be oxybenzone-1 (label name (INCI: Benzophenone-1)), oxybenzone-2 (label name (INCI: Benzophenone-2)), oxybenzone-3 (label name (INCI: Benzophenone-3)), oxybenzone-4 (label name (INCI: Benzophenone-4)), oxybenzone-5 (label name (INCI: Benzophenone-5)), oxybenzone-6 (label name (INCI: Benzophenone-6)), oxybenzone-9 (label name (INCI: Benzophenone-9)), homosalate (INCI), octocrylene (INCI), t-butyl methoxydibenzoylmethane (label name (INCI: Butyl Methoxydibenzoylmethane)), ethylhexyl salicylate (label name (INCI: Ethylhexyl Salicylate)), diethylamino hydroxybenzoyl hexyl benzoate (label name (INCI: Diethylamino Hydroxybenzoyl Hexyl Benzoate)), polysilicone-15 (INCI), octyl dimethoxybenzylidene dioxoimidazolidine propionate (label name (INCI: Ethylhexyl Dimethoxybenzylidene Dioxoimidazolidine Propionate)), terephthalylidene dicamphor sulfonic acid (label name (INCI: Terephthalylidene Dicamphor Sulfonic Acid)), ethylhexyl triazone (INCI), trimethoxycinnamic acid methyl bis(trimethylsiloxy)silyl isopentyl (label name (INCI: Isopentyl Trimethoxycinnamate Trisiloxane)), drometrizole trisiloxane (INCI), dimethyl PABA ethylhexyl (label name (INCI: Ethylhexyl Dimethyl PABA)), isopropyl p-methoxycinnamate (label name (INCI: Isopropyl Methoxycinnamate)), ethylhexyl methoxycinnamate (label name (INCI: Ethylhexyl Methoxycinnamate)), bisethylhexyloxyphenol methoxyphenyl triazine (INCI), phenylbenzimidazole sulfonic acid (label name (INCI: Phenylbenzimidazole Sulfonic Acid)), methylene bisbenzotriazolyl tetramethylbutylphenol (INCI), glyceryl ethylhexanoate dimethoxycinnamate (label name (INCI: Glyceryl Ethylhexanoate Dimethoxycinnamate)), glyceryl PABA (INCI), diisopropyl methyl cinnamate (label name (INCI: Diisopropyl Methyl Cinnamate)), cinoxate (INCI), ethylhexyl dimethoxybenzylidene dioxoimidazolidine propionate (label name (INCI: Ethylhexyl Dimethoxybenzylidene Dioxoimidazolidine Propionate)), or the like. Furthermore, a UVA absorber (for example, diethylamino hydroxybenzoyl hexyl benzoate or the like) and a UVB absorber (for example, ethylhexyl methoxycinnamate or the like) can be used in combination, and each can be arbitrarily combined.

### (2) Surfactant other than (d)

The surfactant is nonionic, anionic, cationic, or amphoteric and may be any surfactant that can be used in ordinary cosmetics.

### (3) Powder

The powder may be a color pigment, an inorganic powder, a metal powder, an organic powder, an inorganic/organic composite powder, or the like. This is more specifically described below.

### • Color Pigment

The color pigment may be any pigment typically used for the purpose of coloring a cosmetic and may be any of color pigments, such as red iron oxide (label name (INCI: Iron Oxides)), yellow iron oxide (label name (INCI: Iron Oxides)), white titanium oxide (label name (INCI: Titanium Dioxide)), black iron oxide (label name (INCI: Iron Oxides)), ultramarine (label name (INCI: Ultramarines)), Prussian blue (label name (INCI: Ferric Ferrocyanide, Ferric Ammonium Ferrocyanide)), manganese violet (label name (INCI: Manganese Violet)), cobalt titanate (label name (INCI: Cobalt Titanium Oxide)), chromium hydroxide (label name (INCI: Chromium Hydroxide Green)), chromium oxide (label name (INCI: Chromium Oxide Greens)), (Al/cobalt) oxide (label name (INCI: Cobalt Aluminum Oxide)), a (titanium/titanium oxide) fired product (label name (INCI: Titanium/Titanium Dioxide)), (Li/cobalt) titanate (label name (INCI: Lithium Cobalt Titanate)), an (iron oxide/titanium oxide) sinter (label name), a composite doped with a different type of metal, such as iron oxide doped titanium oxide (label name (INCI: Iron Oxides, Titanium Dioxide)), titanium nitride (label name (INCI: Titanium Nitride)), iron(II) hydroxide (label name (INCI: Iron Hydroxide)), an inorganic brown pigment, such as γ-iron oxide, an inorganic yellowish pigment, such as ocher, a lake of a tar dye, and a lake of a natural dye. The pigment may have any shape, such as spherical, approximately spherical, rod-like, spindle-like, petal-like, strip-like, or an irregular shape, and may have any geometrical aspect that can impart a color to the cosmetic.

### • Inorganic Powder

The inorganic powder may be fine particles composed of zirconium oxide (label name (INCI: Zirconium Dioxide)), zinc oxide (label name (INCI: Zinc Oxide)), cerium oxide (label name (INCI: Cerium Oxide)), Mg oxide (label name (INCI: Magnesium Oxide)), Ba sulfate (label name (INCI: Barium Sulfate)), calcium sulfate (label name (INCI: Calcium Sulfate)), Mg sulfate (label name (INCI: Magnesium Sulfate)), Ca carbonate (label name (INCI: Calcium Carbonate)), Mg carbonate (label name (INCI: Magnesium Carbonate)), talc (INCI), mica (INCI), kaolin (INCI), synthetic fluorophlogopite (label name (INCI: Synthetic Fluorphlogopite), synthetic phlogopite iron (label name), biotite (label name (INCI: Biotite), K silicate (label name (INCI: Potassium Silicate)), silica (INCI), Al silicate (label name (INCI: Aluminum Silicate)), Mg silicate (label name (INCI: Magnesium Silicate)), (Al/Mg) silicate (label name (INCI: Magnesium Aluminum Silicate)), Ca silicate (label name (INCI: Calcium Silicate)), (Al/Ca/Na) silicate (label name (INCI: Aluminum Calcium Sodium Silicate)), (Li/Mg/Na) silicate (label name (INCI: Lithium Magnesium Sodium Silicate)) (Na/Mg) silicate (label name (INCI: Sodium Magnesium Silicate)), (Ca/Al) borosilicate (label name (INCI: Calcium Aluminum Borosilicate)), (Ca/Na) borosilicate (label name (INCI: Calcium Sodium Borosilicate)), hydroxyapatite (INCI), bentonite (INCI), montmorillonite (INCI), hectorite (INCI), zeolite (INCI), alumina (INCI), Al hydroxide (label name (INCI: Aluminum Hydroxide)), boron nitride (label name (INCI: Boron Nitride)), glass (label name (INCI: Glass)), or the like.

Furthermore, examples of an inorganic color pearl pigment include a pearl, such as mica (INCI) coated with titanium oxide (label name (INCI: Titanium Dioxide)) or synthetic fluorophlogopite (label name (INCI: Synthetic Fluorphlogopite) coated with titanium oxide (label name (INCI: Titanium Dioxide)), and a pearl pigment, such as bismuth oxychloride (label name (INCI: Bismuth Oxychloride)), bismuth oxychloride (label name (INCI: Bismuth Oxychloride)) coated with titanium oxide (label name (INCI: Titanium Dioxide)), talc (INCI) coated with titanium oxide (label name (INCI: Titanium Dioxide)), argentine (label name), or colored mica coated with titanium oxide (label name (INCI: Titanium Dioxide)), and the inorganic color pearl pigment may be untreated or may be subjected to a known surface treatment generally used for cosmetics.

### • Metal Powder

The metal powder may be fine metal particles composed of Al (label name (INCI: Aluminum, Aluminum Powder)), copper (label name (INCI: Copper Powder)), silver (label name (INCI: Silver Powder)), gold (label name (INCI: Gold)), or the like.

### • Organic Powder

The organic powder may be a powder composed of silicone, polyamide, a poly(acrylic acid)-acrylic acid ester, polyester, polyethylene (INCI), polypropylene (INCI), polystyrene (INCI), a styrene-acrylic acid copolymer, a divinylbenzene-styrene copolymer, polyurethane, a vinyl resin, a urea resin, a melamine resin, benzoguanamine, polymethylbenzoguanamine, tetrafluoroethylene, poly(methyl methacrylate), cellulose (INCI), silk (INCI), nylon (label name), a phenolic resin, an epoxy resin, polycarbonate, or the like.

In particular, the silicone may be silicone resin particles; polymethylsilsesquioxane (INCI), a silicone rubber powder, a silicone resin-coated silicone rubber powder; a (vinyl dimethicone/methicone silsesquioxane) crosspolymer (INCI), a (diphenyl dimethicone/vinyl diphenyl dimethicone/silsesquioxane) crosspolymer (INCI), a polysilicone-1 crosspolymer (INCI), polysilicone-22 (INCI), or the like.

Examples of commercially available silicone powders include KMP-590, KMP-591, KMP-592, KMP-597, KMP-598, KSP-100, KSP-101, KSP-102, KSP-105, KSP-300, KSP-411, KSP-441, KM-9729, and KM-440 each manufactured by Shin-Etsu Chemical Co., Ltd.

Furthermore, a metallic soap and the like are also mentioned, and specific examples thereof include a powder composed of zinc stearate (label name (INCI: Zinc Stearate)), Al stearate (label name (INCI: Aluminum Stearate)), Ca stearate (label name (INCI: Calcium Stearate)), Mg stearate (label name (INCI: Magnesium Stearate)), zinc myristate (label name (INCI: Zinc Myristate)), Mg myristate (label name (INCI: Magnesium Myristate)), (zinc/Na) cetyl phosphate (label name (INCI: Sodium Zinc Cetyl Phosphate)), K cetyl phosphate (label name (INCI: Potassium Cetyl Phosphate)), or the like.

Furthermore, an organic dye may also be mentioned, and specific examples thereof include a tar dye, such as red 3, red 104(1) (label name (INCI: Red 28, Red 28 Lake)), red 106, red 201 (label name (INCI: Red 6)), red 202 (label name (INCI: Red 7)), red 204, red 205, red 220 (label name (INCI: Red 34)), red 226 (label name (INCI: Red 30)), red 227 (label name (INCI: Red 33, RED 33 Lake)), red 228 (label name (INCI: Red 36)), red 230(1) (label name (INCI: Red 22, Red 22 Lake)), red 230(2) (label name), red 401 (label name), red 505 (label name), yellow 4 (label name (INCI: Yellow 5)), yellow 5 (label name (INCI: Yellow 6, Yellow 6 Lake)), yellow 202(1) (label name (INCI: Yellow 8)), yellow 203 (label name (INCI: Yellow 10, Yellow 10 Lake)), yellow 204 (label name (INCI: Yellow 11)), yellow 401, blue 1 (label name (INCI: Blue 1, Blue 1 Lake)), blue 2, blue 201, blue 205 (label name (INCI: Blue 4)), blue 404 (label name), green 3 (label name (INCI: Green 3, Green 3 Lake)), green 201 (label name (INCI: Green 5)), green 202 (label name (INCI: Green 6)), green 204 (label name (INCI: Green 8)), green 205 (label name), orange 201 (label name (INCI: Orange 5)), orange 203 (label name (INCI: Pigment Orange 5)), orange 204 (label name), orange 205 (label name (INCI: Orange 4, Orange 4 Lake)), orange 206 (label name (INCI: Orange 10)), or orange 207 (label name (INCI: Orange 11)), and a natural dye, such as cochineal (INCI), laccaic acid (label name (INCI: Laccaic Acid)), safflower red (label name (INCI: Carthamus Tinctorius (Safflower) Flower Extract)), a Lithospermum erythrorhizon root extract (label name (INCI: Lithospermum Officinale Root Extract)), gardenia yellow (label name), or gardenia blue (label name (INCI: Hydrolyzed Gardenia Florida Extract)).

### • Inorganic/Organic Composite Powder

The inorganic/organic composite powder is, for example, a composite powder in which the surface of an inorganic powder is coated with an organic powder by a publicly known method.

The powder may be surface-treated particles. The surface treatment agent is preferably one that can impart hydrophobicity from the perspective of the water resistance of the cosmetic, and the treatment agent that imparts hydrophobicity may be, but is not limited to, a silicone treatment agent, a wax, a paraffin, an organic fluorine compound, such as a perfluoroalkyl and a phosphate, a surfactant, an amino acid, such as N-acylglutamic acid, or a metallic soap, such as aluminum stearate or magnesium myristate. A silicone treatment agent is more preferred and may be a silane or a silylation reagent, such as triethoxycaprylylsilane (INCI) or trimethoxysilyl dimethicone (INCI), dimethicone (INCI), methicone (INCI), hydrogen dimethicone (INCI), triethoxysilylethyl polydimethylsiloxyethyl dimethicone (INCI), triethoxysilylethyl polydimethylsiloxyethyl hexyl dimethicone (INCI), an (acrylates/tridecyl acrylate/triethoxysilylpropyl methacrylate/dimethicone methacrylate) copolymer (label name (INCI: Acrylates/Tridecyl Acrylate/Triethoxysilylpropyl Methacrylate/Dimethicone Methacrylate Copolymer), or the like. In particular, a silicone powder treatment agent described in Japanese Patent No. 3912961 is suitably used.

Specific examples of these silicone treatment agents include AES-3083, KF-99P, KF-9901, KF-9908, KF-9909, KP-574, and KP-541 each manufactured by Shin-Etsu Chemical Co., Ltd. Among them, triethoxysilylethyl polydimethylsiloxyethyl hexyl dimethicone (manufactured by Shin-Etsu Chemical Co., Ltd.: KF-9909), which is a dimethyl polysiloxane having a triethoxysilyl group, a polydimethylsiloxyethyl group, and a hexyl group in a side chain, and the like are preferred from the perspective of exhibiting high affinity even when the dispersion medium for dispersing the powder is silicone, a hydrocarbon, or a mixed composition thereof.

Furthermore, these surface hydrophobizing agents can be used alone or in combination of two or more types thereof. Specific examples of surface-treated color pigments include KTP-09 series manufactured by Shin-Etsu Chemical Co., Ltd., particularly, KTP-09W, 09R, 09Y, 09B, and the like. Specific examples of the hydrophilization treatment include KSP-100W manufactured by Shin-Etsu Chemical Co., Ltd.

### (4) Composition Composed of Cross-Linked Organopolysiloxane other than Silicone Gel in Which Component (a) Is Swollen with Component (b) of Present Invention and Oil Agent That Is Liquid at Room Temperature

A composition composed of a cross-linked organopolysiloxane other than the component (a) and an oil agent that is liquid at room temperature may also be blended. The cross-linked organopolysiloxane preferably contains the liquid oil agent in an amount equal to or greater than its own weight and swells in the liquid oil. As the liquid oil agent, any of the component (b) and (1) a liquid oil in an oil agent as an optional component can be suitably used.

Unlike the cross-linked silicone surfactant of the component (d) described above, the cross-linked organopolysiloxane in the component (4) is a compound without a polyether or polyglycerol structure in the molecular structure, and specific examples thereof include a (dimethicone/vinyl dimethicone) crosspolymer (INCI) and a (dimethicone/phenyl vinyl dimethicone) crosspolymer (INCI).

Examples of commercially available compositions composed of a cross-linked organopolysiloxane and an oil agent that is liquid at room temperature include KSG-15, KSG-1510, KSG-16, KSG-1610, KSG-19, KSG-016F, and KSG-18A each manufactured by Shin-Etsu Chemical Co., Ltd.

### (5) Film-Forming Agent

The film-forming agent is blended mainly for the purpose of further maintaining the effects of the cosmetic. Although there is no particular limitation, a silicone composition is preferred from the perspective of imparting water repellency. More specifically, trimethylsiloxysilicic acid, an acrylic-silicone film agent, silicone-modified norbornene, silicone-modified pullulan, silicone-modified poly(vinyl alcohol), or the like can be used.

Examples of the film-forming agent of the silicone composition include trimethylsiloxysilicic acid (label name (INCI: Trimethylsiloxysilicate)), an (acrylates/dimethicone) copolymer (INCI), a (norbornene/tris(trimethylsiloxy)silylnorbornene) copolymer (INCI), and tri(trimethylsiloxy)silylpropylcarbamic acid pullulan (label name (INCI: Trimethylsiloxysilylcarbamoyl Pullulan)). The film-forming agent may be dissolved in advance in an oil agent that is liquid at room temperature and then blended in the cosmetic. The liquid oil agent may be a liquid silicone oil, hydrocarbon oil, ester oil, natural animal or vegetable oil, semi-synthetic oil, or fluorinated oil. Specific examples of commercially available silicone film-forming agents include KF-7312J, KP-545, KP-549, KP-543, NBN-30-ID, TSPL-30-ID, and TSPL-30-D5 each manufactured by Shin-Etsu Chemical Co., Ltd.

### (6) Ultraviolet Absorber or Scattering Agent

The ultraviolet scattering agent may be particles that absorb and scatter ultraviolet light, such as fine titanium oxide particles, fine iron-containing titanium oxide particles, fine zinc oxide particles, fine cerium oxide particles, or a complex thereof, or may also be a dispersion in which these particles that absorb and scatter ultraviolet light are dispersed in an oil agent in advance.

The oil agent may be (1) a liquid silicone oil, a hydrocarbon oil, an ester oil, a natural animal or vegetable oil, a semi-synthetic oil, or a fluorinated oil in the oil agent as an optional component.

Specific examples of the dispersion produced by dispersing the particles that absorb and scatter ultraviolet light in an oil agent in advance include SPD series (trade name) manufactured by Shin-Etsu Chemical Co., Ltd., particularly, SPD-T5, T5L, Z5, Z5L, T6, Z6, T7, and Z7L.

### (7) Other Additive Agents

Examples of other additive agents include an oil-soluble gelling agent, a preservative/bactericide, an antiperspirant, a perfume, a salt, an antioxidant, a pH adjuster, a chelating agent, a refreshing agent, an anti-inflammatory agent, a skin-beautifying component (whitening agent, cell activator, skin roughness improving agent, blood circulation promoter, skin astringent, antiseborrheic agent, or the like), a vitamin, an amino acid, a nucleic acid, a hormone, and an inclusion compound.

### • Oil-Soluble Gelling Agent

The oil-soluble gelling agent may be a metallic soap, such as aluminum stearate, magnesium stearate, or zinc myristate; an amino acid derivative, such as lauroyl glutamic acid (label name (INCI: Lauroyl Glutamic Acid)) or α,γ-di-n-butylamine; a dextrin fatty acid ester, such as dextrin palmitate (label name (INCI: Dextrin Palmitate)), dextrin isostearate (label name (INCI: Dextrin Isostearate)), dextrin myristate (label name (INCI: Dextrin Myristate)), inulin stearate (label name (INCI: Stearoyl Inulin)), or dextrin (palmitate/ethylhexanoate) (label name (INCI: Dextrin Palmitate/Ethylhexanoate)); a sucrose fatty acid ester, such as sucrose palmitate or sucrose stearate; a fructo oligosaccharide fatty acid ester, such as fructo oligosaccharide stearate or fructo oligosaccharide 2-ethylhexanoate; a benzylidene derivative of sorbitol, such as monobenzylidene sorbitol or dibenzylidene sorbitol; an organic modified clay mineral, such as disteardimonium hectorite (INCI), stearalkonium hectorite, or hectorite; stearalkonium bentonite (INCI); or the like.

### • Preservative/Bactericide

The preservative/bactericide may be an alkyl p-hydroxybenzoate, benzoic acid, sodium benzoate, sorbic acid, potassium sorbate, phenoxyethanol, imidazolidinyl urea, salicylic acid, isopropylmethylphenol, phenol, p-chloro-m-cresol, hexachlorophene, benzalkonium chloride, chlorhexidine hydrochloride, trichlorocarbanilide, iodopropynyl butylcarbamate, polylysine, a photosensitive substance, silver, a plant extract, or the like.

### • Antiperspirant

The antiperspirant may be an aluminum hydroxyhalide, such as AL chlorohydrate, an aluminum halide, such as AL chloride, an allantoin aluminum salt, tannic acid, persimmon tannin, (AL/K) sulfate, zinc oxide, zinc p-phenolsulfonate, burnt alum, tetrachloro(Al/zirconium) hydrate, trichlorohydrex glycine (Al/zirconium), or the like.

In particular, an aluminum hydroxyhalide, an aluminum halide, a complex or mixture thereof with a zirconyl oxyhalide or a zirconyl hydroxyhalide (for example, tetrachloro(Al/zirconium) hydrate or trichlorohydrex glycine (Al/zirconium)), or the like is preferred as a component that exhibits high effects.

### • Perfume

The perfume is a natural perfume or a synthetic perfume. The natural perfume is a plant perfume separated from a flower, leave, wood, pericarp, or the like; or an animal perfume, such as musk or civet. The synthetic perfume may be a hydrocarbon, such as monoterpene; an alcohol, such as an aliphatic alcohol or an aromatic alcohol; an aldehyde, such as a terpene aldehyde or an aromatic aldehyde; a ketone, such as an alicyclic ketone; an ester, such as a terpene ester; a lactone; a phenol; an oxide; a nitrogen-containing compound; an acetal; or the like.

### • Salt

The salt may be an inorganic salt, an organic acid salt, an amine salt, or an amino acid salt. The inorganic salt is, for example, a sodium salt, a potassium salt, a magnesium salt, a calcium salt, an aluminum salt, a zirconium salt, or a zinc salt of an inorganic acid, such as hydrochloric acid, sulfuric acid, carbonic acid, or nitric acid; the organic acid salt is, for example, a salt of an organic acid, such as acetic acid, dehydroacetic acid, citric acid, malic acid, succinic acid, ascorbic acid, or stearic acid; and the amine salt or an amino acid salt is, for example, a salt of an amine, such as triethanolamine, or a salt of an amino acid, such as glutamic acid. Furthermore, a salt, such as hyaluronic acid or chondroitin sulfate, an aluminum zirconium glycine complex, and an acid-alkali neutralized salt used in cosmetic formulations can also be used. When a salt is blended, the blending amount thereof preferably ranges from 0.1% to 3.0% by mass, preferably 0.5% to 2.5% by mass, preferably 1.0% to 2.0% by mass, of the entire cosmetic. Among these, in particular, Na chloride (label name (INCI: Sodium Chloride) and Mg sulfate (label name (INCI: Magnesium Sulfate) are easy to handle and easy to blend in the formulation, and Mg sulfate is more preferred from the perspective of high stabilization effect.

### • Antioxidant

The antioxidant is, for example, but not limited to, a carotenoid, ascorbic acid or a salt thereof, ascorbyl stearate, tocopherol, tocopherol acetate, tocopherol, p-t-butylphenol, butylated hydroxyanisole, butylated hydroxytoluene, phytic acid, ferulic acid, thiotaurine, hypotaurine, sulfite, erythorbic acid or a salt thereof, chlorogenic acid, epicatechin, epigallocatechin, epigallocatechin gallate, apigenin, kaempferol, myricetin, quercetin, or the like. The antioxidant can be used alone or in combination of two or more types thereof.

### • pH Adjuster

The pH adjuster may be lactic acid, citric acid, glycolic acid, succinic acid, tartaric acid, dl-malic acid, potassium carbonate, sodium hydrogen carbonate, ammonium hydrogen carbonate, or the like.

### • Chelating Agent

The chelating agent may be alanine, sodium edetate, sodium polyphosphate, sodium metaphosphate, phosphoric acid, or the like.

### • Refreshing Agent

The refreshing agent may be L-menthol, camphor, menthyl lactate, or the like.

### • Anti-Inflammatory Agent

The anti-inflammatory agent may be allantoin, glycyrrhizic acid or a salt thereof, glycyrrhetinic acid, stearyl glycyrrhetinate, tranexamic acid, azulene, or the like.

### • Skin-Beautifying Component

The skin-beautifying component may be a whitening agent, such as a placenta extract, arbutin, glutathione, or a saxifrage extract, a wrinkle-improving agent, such as retinol or niacinamide, a cell activator, such as royal jelly, a photosensitive substance, a cholesterol derivative, or a calf blood extract; a skin roughness improving agent, a blood circulation promoter, such as nonivamide, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, or γ-oryzanol, a skin astringent, such as zinc oxide or tannic acid, an antiseborrheic agent, such as sulfur or thianthol, or the like.

### • Vitamin

The vitamin may be vitamin A, such as vitamin A oil, retinol acetate, or retinol palmitate, vitamin B, such as vitamin B2, such as riboflavin, riboflavin butyrate, or flavin adenine nucleotide, vitamin B6, such as pyridoxine hydrochloride, pyridoxine dioctanoate, or pyridoxine tripalmitate, vitamin B12 or a derivative thereof, or vitamin B15 or a derivative thereof, vitamin C, such as L-ascorbic acid, L-ascorbic acid dipalmitate, L-ascorbic acid-2-sodium sulfate, or L-ascorbic acid phosphoric diester dipotassium, vitamin D, such as ergocalciferol or cholecalciferol, vitamin E, such as α-tocopherol, β-tocopherol, γ-tocopherol, dl-α-tocopherol acetate, dl-α-tocopherol nicotinate, or dl-α-tocopherol succinate; nicotinic acid, such as nicotinic acid, benzyl nicotinate, or nicotinamide, vitamin H, vitamin P, a pantothenic acid, such as calcium pantothenate, D-pantothenyl alcohol, pantothenyl ethyl ether, or acetylpantothenyl ethyl ether, biotin, or the like.

### • Amino Acid

The amino acid may be glycine, valine, leucine, isoleucine, serine, threonine, phenylalanine, arginine, lysine, aspartic acid, glutamic acid, cystine, cysteine, methionine, tryptophan, or the like.

### • Nucleic Acid

The nucleic acid may be deoxyribonucleic acid or the like.

### • hormone

The hormone may be estradiol, ethenyl estradiol, or the like.

### • Inclusion Compound

The inclusion compound may be cyclodextrin or the like.

### EXAMPLES

Although the present invention will be more specifically described with reference to Production Examples, Comparative Production Examples, and Examples and Comparative Examples of cosmetics, the present invention is not limited to these Examples. Unless otherwise specified, "%" in the composition described below means "% by mass", the mass percentage of each component is based on the total mass (100% by mass) in each example, and the blending amount is the blending amount of the blended product described. Siloxane units in the structural formulae may be bonded in any order.

### [Production Example 1]

280 g of an organohydrogen polysiloxane with an alkyl branched chain represented by the following structural formula (1), 185 g of an organopolysiloxane with a vinyl group on both ends represented by the following structural formula (2), and 0.1 g of 2% chloroplatinic acid in ethanol were charged into a reaction vessel and were polymerized at 80°C for 1 hour to prepare a cross-linked silicone. 35 g of the cross-linked silicone as the component (a) was kneaded with 65 g of a cocoalkyl (caprylate/caprate) (IOB = 0.15, kinematic viscosity = 5 mm²/s) as the component (b) to prepare a silicone gel. The resulting composition had a total light transmittance of 91% and a haze of 51%.

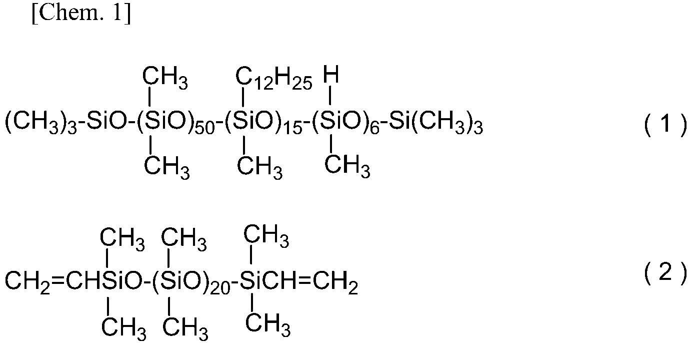

### [Production Example 2]

500 g of KSG-42A (a mixture of isododecane 75% to 85% + (vinyl dimethicone/lauryl dimethicone) crosspolymer 15% to 25% manufactured by Shin-Etsu Chemical Co., Ltd.) was charged into a reaction vessel, and the solvent was removed under reduced pressure at 120°C. 25 g of the resulting cross-linked silicone as the component (a) was kneaded with 75 g of a cocoalkyl (caprylate/caprate) (IOB = 0.15, kinematic viscosity = 5 mm²/s) as the component (b) to prepare a silicone gel. The silicone gel had a total light transmittance of 93% and a haze of 3%.

### [Production Example 3]

35 g of the cross-linked silicone produced in Production Example 1 as the component (a) and 65 g of undecane (boiling point = 195°C, kinematic viscosity = 1.6 mm²/s) as the component (b) were kneaded to prepare a silicone gel. The silicone gel had a total light transmittance of 89% and a haze of 65%.

### [Production Example 4]

35 g of the cross-linked silicone produced in Production Example 1 as the component (a) and 65 g of tridecane (boiling point = 235°C, kinematic viscosity = 2.3 mm²/s) as the component (b) were kneaded to prepare a silicone gel. The silicone gel had a total light transmittance of 90% and a haze of 59%.

### [Production Example 5]

25 g of the cross-linked silicone produced in Production Example 2 as the component (a) and 75 g of a C13-15 alkane (boiling point = 246°C, kinematic viscosity = 2.5 mm²/s) as the component (b) were kneaded to prepare a silicone gel. The silicone gel had a total light transmittance of 92% and a haze of 4%.

### [Production Example 6]

25 g of the cross-linked silicone produced in Production Example 2 as the component (a) and 75 g of (caprylic/capric) triglyceride (IOB = 0.3, kinematic viscosity = 23 mm²/s) as the component (b) were kneaded to prepare a silicone gel. The silicone gel had a total light transmittance of 93% and a haze of 3%.

### [Production Example 7]

500 g of KSG-045Z (a mixture of cyclopentasiloxane75% to 85% + (lauryl polydimethylsiloxyethyl dimethicone/bisvinyl dimethicone) crosspolymer 15% to 25% manufactured by Shin-Etsu Chemical Co., Ltd.) was charged into a reaction vessel, and the solvent was removed under reduced pressure at 120°C. 25 g of the resulting cross-linked silicone as the component (a) and 75 g of a cocoalkyl (caprylate/caprate) (IOB = 0.15, kinematic viscosity = 4 mm²/s) as the component (b) were kneaded to prepare a silicone gel. The silicone gel had a total light transmittance of 91% and a haze of 45%.

The advantages of the present invention will be described using Examples and Comparative Examples relating to cosmetics. Production Examples and Comparative Production Examples represent silicone gels produced in the respective Examples.

### [Examples 1 to 7, Comparative Examples 1 to 5]

Non-aqueous cosmetics having the formulations shown in Table 1 were prepared. The cosmetics were evaluated as described below.

### (Production Method)

The component (2) was dispersed and mixed in the component (1) to prepare a non-aqueous cosmetic.

### [Property Evaluation]

The usability (less stickiness) and emollient feel (less dry feel) of cosmetics were evaluated by 10 expert panelists according to the following evaluation criteria. The results were evaluated according to the following determination criteria based on the average value of the 10 panelists. The results are also shown in the table.

### [Evaluation Criteria]

| | |
|---|---|
| 5 points: | very good |
| 4 points: | good |
| 3 points: | fair |
| 2 points: | slightly poor |
| 1 point: | poor |

### [Determination Criteria]

| | |
|---|---|
| A: | average score of 4.5 or more |
| B: | average score of 3.5 or more and less than 4.5 |
| C: | average score of 2.5 or more and less than 3.5 |
| D: | average score of 1.5 or more and less than 2.5 |
| E: | average score of less than 1.5 |

"C" or higher (A, B, and C) was regarded as acceptable.

**[Table 1]**

| Composition (%) | | Example | | | | | | | Comparative Example | | | | |
|---|---|---|---|---|---|---|---|---|---|---|---|---|---|
| | | 1 | 2 | 3 | 4 | 5 | 6 | 7 | 1 | 2 | 3 | 4 | 5 |
| | Production Example 1 | 91 | | | | | | | | | | | |
| | Production Example 2 | | 91 | | | | | | | | | | |
| | Production Example 3 | | | 91 | | | | | | | | | |
| | Production Example 4 | | | | 91 | | | | | | | | |
| | Production Example 5 | | | | | 91 | | | | | | | |
| (1) | Production Example 6 | | | | | | 91 | | | | | | |
| | Production Example 7 | | | | | | | 91 | | | | | |
| | KSG-41A (Note 1) | | | | | | | | 91 | | | | |
| | KSG-42A (Note 2) | | | | | | | | | 91 | | | |
| | KSG-43 (Note 3) | | | | | | | | | | 91 | | |
| | KSG-44 (Note 4) | | | | | | | | | | | 91 | |
| | KSG-045Z (Note 5) | | | | | | | | | | | | 91 |
| (2) | KMP-590 (Note 6) | 9 | 9 | 9 | 9 | 9 | 9 | 9 | 9 | 9 | 9 | 9 | 9 |
| Total | | 100 | 100 | 100 | 100 | 100 | 100 | 100 | 100 | 100 | 100 | 100 | 100 |
| Usability | | A | B | A | A | A | C | A | D | A | D | D | A |
| Emollient feel | | A | A | C | B | B | A | A | A | D | A | A | E |

| | | | | | | | | | | | | | |
|---|---|---|---|---|---|---|---|---|---|---|---|---|---|
| (Note 1) A mixture of mineral oil (kinematic viscosity = 15.1 mm²/s) 70% to 80% + (vinyl dimethicone/lauryl dimethicone) crosspolymer 20% to 30% manufactured by Shin-Etsu Chemical Co., Ltd. (Note 2) A mixture of isododecane (boiling point = 179°C to 182°C) 75% to 85% + (vinyl dimethicone/lauryl dimethicone) crosspolymer 15% to 25% manufactured by Shin-Etsu Chemical Co., Ltd. (Note 3) A mixture of triethylhexanoin (IOB = 0.35, kinematic viscosity = 30 mm²/s) 65% to 75% + (vinyl dimethicone/lauryl dimethicone) crosspolymer 25% to 35% manufactured by Shin-Etsu Chemical Co., Ltd. (Note 4) A mixture of squalane (kinematic viscosity = 39 mm²/s) 65% to 75% + (vinyl dimethicone/lauryl dimethicone) crosspolymer 25% to 35% manufactured by Shin-Etsu Chemical Co., Ltd. (Note 5) A mixture of cyclopentasiloxane 75% to 85% + (lauryl polydimethylsiloxyethyl dimethicone/bisvinyl dimethicone) crosspolymer 15% to 25% manufactured by Shin-Etsu Chemical Co., Ltd. (Note 6) Polymethylsilsesquioxane manufactured by Shin-Etsu Chemical Co., Ltd. | | | | | | | | | | | | | |

As is clear from Table 1, it was found that the cosmetics of Examples 1 to 7 had high usability and a good emollient feel. On the other hand, Comparative Examples 1 to 5, in which the component (b) of the present invention was not used, had poor usability or a poor emollient feel.

### [Examples 8 to 10, Comparative Examples 6 to 10]

Water-in-oil (W/O) cosmetics having the formulations shown in Table 2 were prepared. The cosmetics were evaluated as described below.

### (Production Method)

A: The component (1) was mixed.
B: The component (2) was mixed.
C: The mixture prepared in B was added to the mixture prepared in A and was emulsified to prepare a water-in-oil (W/O) cosmetic.

### [Property Evaluation]

The usability (less stickiness), emollient feel (less dry feel), and soft focus effect (inconspicuous pores and wrinkles) of cosmetics were evaluated by 10 expert panelists according to the following evaluation criteria. The results were evaluated according to the following determination criteria based on the average value of the 10 panelists. The results are also shown in the table.

### [Evaluation Criteria]

| | |
|---|---|
| 5 points: | very good |
| 4 points: | good |
| 3 points: | fair |
| 2 points: | slightly poor |
| 1 point: | poor |

### [Determination Criteria]

| | |
|---|---|
| A: | average score of 4.5 or more |
| B: | average score of 3.5 or more and less than 4.5 |
| C: | average score of 2.5 or more and less than 3.5 |
| D: | average score of 1.5 or more and less than 2.5 |
| E: | average score of less than 1.5 |

"B" or higher (A and B) was regarded as acceptable.

### [Physical Properties of Silicone Gel]

Production Examples and Comparative Production Examples each having a thickness of 500 µm were measured on a silica glass plate with a thickness of 1 mm. A haze meter (HR-100 type) manufactured by Murakami Color Research Laboratory was used as a measuring instrument. The total light transmittance is measured according to JIS K 7361, and the haze is measured according to JIS K 7136.

**[Table 2]**

| Composition (%) | | | Example | | | Comparative Example | | | | |
|---|---|---|---|---|---|---|---|---|---|---|
| | | | 8 | 9 | 10 | 6 | 7 | 8 | 9 | 10 |
| (1) | Production Example 1 | | 30 | | | | | | | |
| | Production Example 3 | | | 30 | | | | | | |
| | Production Example 4 | | | | 30 | | | | | |
| | KSG-41A (Note 1) | | | | | 30 | | | | |
| | KSG-42A (Note 2) | | | | | | 30 | | | |
| | KSG-43 (Note 3) | | | | | | | 30 | | |
| | KSG-44 (Note 4) | | | | | | | | 30 | |
| | KSG-045Z (Note 5) | | | | | | | | | 30 |
| (2) | KSG-840 (Note 6) | | 3 | 3 | 3 | 3 | 3 | 3 | 3 | 3 |
| | KF-6105 (Note 7) | | 1.3 | 1.3 | 1.3 | 1.3 | 1.3 | 1.3 | 1.3 | 1.3 |
| | Jojoba seed oil | | 10 | 10 | 10 | 10 | 10 | 10 | 10 | 10 |
| (3) | BG | | 5 | 5 | 5 | 5 | 5 | 5 | 5 | 5 |
| | Glycerol | | 3 | 3 | 3 | 3 | 3 | 3 | 3 | 3 |
| | Citric acid Na | | 0.2 | 0.2 | 0.2 | 0.2 | 0.2 | 0.2 | 0.2 | 0.2 |
| | Na chloride | | 0.5 | 0.5 | 0.5 | 0.5 | 0.5 | 0.5 | 0.5 | 0.5 |
| | Water | | 47 | 47 | 47 | 47 | 47 | 47 | 47 | 47 |
| Total | | | 100 | 100 | 100 | 100 | 100 | 100 | 100 | 100 |
| Physical properties of (1) | | Transmittance (%) | 91 | 89 | 90 | 93 | 92 | 93 | 93 | 91 |
| | | Haze (%) | 51 | 65 | 59 | 8 | 21 | 3 | 8 | 35 |
| Usability | | | A | A | A | C | A | C | C | A |
| Emollient feel | | | A | B | B | A | C | A | A | D |
| Soft focus effect | | | B | A | A | E | D | E | E | C |

| | | | | | | | | | | |
|---|---|---|---|---|---|---|---|---|---|---|
| (Note 1) A mixture of mineral oil (kinematic viscosity = 17 mm²/s) 70% to 80% + (vinyl dimethicone/lauryl dimethicone) crosspolymer 20% to 30% manufactured by Shin-Etsu Chemical Co., Ltd. (Note 2) A mixture of isododecane (boiling point = 177°C, kinematic viscosity = 1.4 mm²/s) 75% to 85% + (vinyl dimethicone/lauryl dimethicone) crosspolymer 15% to 25% manufactured by Shin-Etsu Chemical Co., Ltd. (Note 3) A mixture of triethylhexanoin (IOB = 0.35, kinematic viscosity = 30 mm²/s) 65% to 75% + (vinyl dimethicone/lauryl dimethicone) crosspolymer 25% to 35% manufactured by Shin-Etsu Chemical Co., Ltd. (Note 4) A mixture of squalane (kinematic viscosity = 30 mm²/s) 65% to 75% + (vinyl dimethicone/lauryl dimethicone) crosspolymer 25-35% manufactured by Shin-Etsu Chemical Co., Ltd. (Note 5) A mixture of cyclopentasiloxane 75% to 85% + (lauryl polydimethylsiloxyethyl dimethicone/bisvinyl dimethicone) crosspolymer 15% to 25% manufactured by Shin-Etsu Chemical Co., Ltd. (Note 6) A mixture of squalane 65% to 75% + (lauryl dimethicone/polyglycerol-3) crosspolymer 25% to 35% manufactured by Shin-Etsu Chemical Co., Ltd. (Note 7) Lauryl polyglyceryl-3 polydimethylsiloxyethyl dimethicone manufactured by Shin-Etsu Chemical Co., Ltd. | | | | | | | | | | |

As is clear from Table 2, it was found that the cosmetics of Examples 8 to 10 had high usability, a good emollient feel, and a good soft focus effect. On the other hand, Comparative Examples 6 to 10, in which the component (b) of the present invention was not used, were inferior in any of the usability, emollient feel, and soft focus effect.

### [Example 11, Comparative Example 11]

Oil-in-water (O/W) cosmetics having the formulations shown in Table 3 were prepared. The cosmetics were evaluated as described below.

### (Production Method)

A: The component (1) was mixed.
B: The component (2) was mixed.
C: The mixture prepared in B was added to the mixture prepared in A and was emulsified to prepare a cosmetic.

### [Property Evaluation]

The usability (less stickiness) and soft focus effect (inconspicuous pores and wrinkles) of cosmetics were evaluated by 10 expert panelists according to the following evaluation criteria. The results were evaluated according to the following determination criteria based on the average value of the 10 panelists. The results are also shown in the table.

### [Evaluation Criteria]

| | |
|---|---|
| 5 points: | very good |
| 4 points: | good |
| 3 points: | fair |
| 2 points: | slightly poor |
| 1 point: | poor |

### [Determination Criteria]

| | |
|---|---|
| A: | average score of 4.5 or more |
| B: | average score of 3.5 or more and less than 4.5 |
| C: | average score of 2.5 or more and less than 3.5 |
| D: | average score of 1.5 or more and less than 2.5 |
| E: | average score of less than 1.5 |

"B" or higher (A and B) was regarded as acceptable.

### [Physical Properties of Production Examples]

Production Examples and Comparative Production Examples each having a thickness of 500 µm were measured on a silica glass plate with a thickness of 1 mm. A haze meter (HR-100 type) manufactured by Murakami Color Research Laboratory was used as a measuring instrument. The total light transmittance is measured according to JIS K 7361, and the haze is measured according to JIS K 7136.

**[Table 3]**

| Composition (%) | | | Example | Comparative Example |
|---|---|---|---|---|
| | | | 11 | 11 |
| (1) | Production Example 1 (component (a) 35%) | | 8.6 | |
| | KSG-43 (component (a) 30%) (Note 1) | | | 10 |
| | (b) Cocoalkyl (caprylate/caprate) | | 1.4 | 6.5 |
| | Triethylhexanoin | | 6.5 | |
| (2) | BG | | 10 | 10 |
| | KF-6043 (Note 2) | | 1.5 | 1.5 |
| | SEPIGEL 305 (Note 3) | | 1.5 | 1.5 |
| | Water | | 70.5 | 70.5 |
| Total | | | 100 | 100 |
| Physical properties of Production Example | | Transmittance (%) | 91 | 93 |
| | | Haze (%) | 51 | 8 |
| Usability | | | B | D |
| Soft focus effect | | | B | E |

| | | | | |
|---|---|---|---|---|
| (Note 1) A mixture of triethylhexanoin (IOB = 0.35, kinematic viscosity = 30 mm²/s) 65% to 75% + (vinyl dimethicone/lauryl dimethicone) crosspolymer 25% to 35% manufactured by Shin-Etsu Chemical Co., Ltd. (Note 2) PEG-10 dimethicone manufactured by Shin-Etsu Chemical Co., Ltd. (Note 3) A polyacrylamide mixture manufactured by SEPPIC | | | | |

As is clear from Table 3, it was found that the cosmetic of Example 11 had high usability and a good soft focus effect. In particular, Comparative Example 11, in which the amount of the oil agent in the oil phase was the same, had insufficient performance. In the silicone gel of KSG-43 (the component (a) 30%), the component (a) is swollen with an oil agent other than the component (b) of the present invention, and it is not a silicone gel in which the component (a) is swollen in advance with the component (b). In Comparative Example 11 in which the component (b) was blended in the same amount as in Example 11, it was also found that the intended effects were not produced, and the advantages of the present invention therefore cannot be achieved by simply blending the component (b) in the cosmetic.

### [Example 12] Water-in-Oil Lipstick

| | Composition | % |
|---|---|---|
| 1. | KF-56A (Note 1) | 11.2 |
| 2. | KF-6105 (Note 2) | 3 |
| 3. | Production Example 1 | 1 |
| 4. | KF-6048 (Note 3) | 1 |
| 5. | KSP-441 (Note 4) | 3 |
| 6. | Ceresin | 5 |
| 7. | Inulin stearate | 3 |
| 8. | Hydrogenated polyisobutene | 5 |
| 9. | (Caprylic/capric) triglyceride | 3 |
| 10. | Neopentyl glycol dicaprate | 2 |
| 11. | Mineral oil | 3 |
| 12. | Sorbitan sesquiisostearate | 0.5 |
| 13. | Red 201 | 0.4 |
| 14. | Red 202 | 0.4 |
| 15. | Yellow 4 | 1.6 |
| 16. | KTP-09W (Note 5) | 3 |
| 17. | KTP-09R (Note 5) | 0.7 |
| 18. | KTP-09B (Note 5) | 0.2 |
| 19. | KP-574 (Note 6) treated talc | 0.7 |
| 20. | Glycerol | 10 |
| 21. | Pentylene glycol | 3 |
| 22. | Ethylhexyl glycerol | 0.1 |
| 23. | Purified water | 40 |
| | Total | 100.0 |

| | | |
|---|---|---|
| (Note 1) Diphenylsiloxyphenyl trimethicone manufactured by Shin-Etsu Chemical Co., Ltd. (Note 2) Lauryl polyglyceryl-3 polydimethylsiloxyethyl dimethicone manufactured by Shin-Etsu Chemical Co., Ltd. (Note 3) Cetyl PEG/PPG-10/1 dimethicone manufactured by Shin-Etsu Chemical Co., Ltd. (Note 4) Polysilicone-22 manufactured by Shin-Etsu Chemical Co., Ltd. (Note 5) KF-9909-treated color inorganic pigments W: white, R: red, Y: yellow, B: black each manufactured by Shin-Etsu Chemical Co., Ltd. (Note 6) An (acrylates/tridecyl acrylate/triethoxysilylpropyl methacrylate/dimethicone methacrylate) copolymer manufactured by Shin-Etsu Chemical Co., Ltd. | | |

### (Production Method)

A: The components 10 to 19 were dispersed with a three-roll mill.
B: The components 1 to 9 were uniformly mixed at 95°C.
C: The components 20 to 23 were uniformly mixed at 85°C.
D: The mixture prepared in A was added to the mixture prepared in B, and C was added thereto at 85°C, was emulsified, was poured into a stick container, and was cooled to prepare a water-in-oil lipstick.

The water-in-oil lipstick was a cosmetic with an emollient feel, high usability, and a good soft focus effect.

### [Example 13] Water-in-Oil Sunscreen Cream

| | Composition | % |
|---|---|---|
| 1. | KSG-820Z (Note 1) | 3 |
| 2. | Production Example 2 | 3 |
| 3. | KF-6105 (Note 2) | 1.5 |
| 4. | KF-56A (Note 3) | 11 |
| 5. | Ethylhexyl methoxycinnamate | 7 |
| 6. | KSP-100 (Note 4) | 2 |
| 7. | Xanthan gum | 0.3 |
| 8. | Dipropylene glycol | 5 |
| 9. | Glycerol | 3 |
| 10. | Methylparaben | 0.1 |
| 11. | Sodium citrate | 0.2 |
| 12. | Sodium chloride | 0.5 |
| 13. | Water | 63.4 |
| | Total | 100.0 |

| | | |
|---|---|---|
| (Note 1) A mixture of isododecane 70% to 80% + (polyglycerol-3/lauryl polydimethylsiloxyethyl dimethicone) crosspolymer 20% to 30% manufactured by Shin-Etsu Chemical Co., Ltd. (Note 2) Lauryl polyglyceryl-3 polydimethylsiloxyethyl dimethicone manufactured by Shin-Etsu Chemical Co., Ltd. (Note 3) Diphenylsiloxyphenyl trimethicone manufactured by Shin-Etsu Chemical Co., Ltd. (Note 4) A (vinyl dimethicone/methicone silsesquioxane) crosspolymer manufactured by Shin-Etsu Chemical Co., Ltd. | | |

### (Production Method)

A: The components 1 to 6 were uniformly mixed.
B: The components 7 to 13 were uniformly mixed.
C: The mixture prepared in B was added to the mixture prepared in A and was emulsified to prepare a water-in-oil sunscreen cream.

The water-in-oil sunscreen cream was a cosmetic with an emollient feel, high usability, and a good soft focus effect.

### [Example 14] W/O Cosmetic Stick

| | Composition | % |
|---|---|---|
| 1. | KSG-330 (Note 1) | 5 |
| 2. | Production Example 3 | 5 |
| 3. | KF-6105 (Note 2) | 0.5 |
| 4. | Meadowfoam oil | 2 |
| 5. | KF-56A (Note 3) | 8 |
| 6. | Argania spinosa kernel oil | 0.5 |
| 7. | Ceresin | 10 |
| 8. | BG | 7 |
| 9. | PCA-Na | 2 |
| 10. | Diglycerol | 3 |
| 11. | Na citrate | 0.2 |
| 12. | Mg sulfate | 1 |
| 13. | Water | 55.8 |
| | Total | 100.0 |

| | | |
|---|---|---|
| (Note 1) A mixture of triethylhexanoin 75% to 85% + (PEG-15/lauryl dimethicone) crosspolymer 15% to 25% manufactured by Shin-Etsu Chemical Co., Ltd. (Note 2) Lauryl polyglyceryl-3 polydimethylsiloxyethyl dimethicone manufactured by Shin-Etsu Chemical Co., Ltd. (Note 3) Diphenylsiloxyphenyl trimethicone manufactured by Shin-Etsu Chemical Co., Ltd. | | |

### (Production Method)

A: The components 1 to 7 were uniformly mixed at 90°C.
B: The components 8 to 13 were uniformly mixed at 85°C.
C: The mixture prepared in B was added to the mixture prepared in A, was emulsified, and was charged into a stick container to prepare a W/O cosmetic stick.

The W/O cosmetic stick was a cosmetic with an emollient feel, high usability, and a good soft focus effect.

### [Example 15] Balm

| | Composition | % |
|---|---|---|
| 1. | Vaseline | 27 |
| 2. | Squalane | 25.8 |
| 3. | KSP-101 (Note 1) | 10 |
| 4. | KMP-590 (Note 2) | 6 |
| 5. | Production Example 6 | 5 |
| 6. | KSG-43 (Note 3) | 3 |
| 7. | Dimethicone (6 cs) | 7 |
| 8. | KF-56A (Note 4) | 4 |
| 9. | KP-561P (Note 5) | 2 |
| 10. | KF-6038 (Note 6) | 0.2 |
| 11. | Dextrin palmitate | 10 |
| | Total | 100.0 |

| | | |
|---|---|---|
| (Note 1) A (vinyl dimethicone/methicone silsesquioxane) crosspolymer manufactured by Shin-Etsu Chemical Co., Ltd. (Note 2) Polymethylsilsesquioxane manufactured by Shin-Etsu Chemical Co., Ltd. (Note 3) A mixture of triethylhexanoin 65% to 75% + (vinyl dimethicone/lauryl dimethicone) crosspolymer 25% to 35% manufactured by Shin-Etsu Chemical Co., Ltd. (Note 4) Diphenylsiloxyphenyl trimethicone manufactured by Shin-Etsu Chemical Co., Ltd. (Note 5) An (acrylates/stearyl acrylate/dimethicone methacrylate) copolymer manufactured by Shin-Etsu Chemical Co., Ltd. (Note 6) Lauryl PEG-9 polydimethylsiloxyethyl dimethicone manufactured by Shin-Etsu Chemical Co., Ltd. | | |

### (Production Method)

The components 1 to 11 were uniformly mixed at 90°C and were cooled to prepare a balm.

The balm was a cosmetic with an emollient feel, high usability, and a good soft focus effect.

### [Example 16] O/W Sunscreen Cream

| | Composition | % |
|---|---|---|
| 1. | Hydroxyethyl cellulose | 0.1 |
| 2. | Ethanol | 10 |
| 3. | BG | 6 |
| 4. | Allantoin | 0.1 |
| 5. | SIMULGEL EG (Note 1) | 2.5 |
| 6. | Water | 59.25 |
| 7. | KF-56A (Note 2) | 3 |
| 8. | Production Example 5 | 1 |
| 9. | Cetanol | 2 |
| 10. | Ethylhexyl methoxycinnamate | 7.5 |
| 11. | Diethylamino hydroxybenzoyl hexyl benzoate | 2 |
| 12. | Ethylhexyl salicylate | 5 |
| 13. | Polyoxyethylene (60) hydrogenated castor oil | 1 |
| 14. | KF-6011 (Note 3) | 0.5 |
| 15. | Tocopherol | 0.05 |
| | Total | 100.0 |

| | | |
|---|---|---|
| (Note 1) A sodium acrylate-sodium acryloyldimethyltaurate copolymer composition manufactured by SEPPIC (Note 2) Diphenylsiloxyphenyl trimethicone manufactured by Shin-Etsu Chemical Co., Ltd. (Note 3) PEG-11 methyl ether dimethicone manufactured by Shin-Etsu Chemical Co., Ltd. | | |

### (Production Method)

A: The components 1 to 6 were heated to 85°C and were uniformly mixed.
B: The components 7 to 15 were heated to 85°C and were uniformly mixed.
C: The mixture prepared in B was added to the mixture prepared in A, was emulsified at 85°C, and was then gradually cooled with stirring to prepare an O/W sunscreen cream.

The O/W sunscreen cream was a cosmetic with an emollient feel, high usability, and a good soft focus effect.

### [Example 17] Sunscreen Gel

| | Composition | % |
|---|---|---|
| 1. | KSG-42A (Note 1) | 10 |
| 2. | Production Example 4 | 35 |
| 3. | KF-4422 (Note 2) | 2.5 |
| 4. | KF-56A (Note 3) | 5 |
| 5. | Octocrylene | 2 |
| 6. | Homosalate | 10 |
| 7. | Ethylhexyl salicylate | 5 |
| 8. | Bisethylhexyloxyphenol methoxyphenyl triazine | 3 |
| 9. | KSP-441 (Note 4) | 10 |
| 10. | KMP-592 (Note 5) | 2 |
| 11. | Production Example 7 | 15.5 |
| | Total | 100.0 |

| | | |
|---|---|---|
| (Note 1) A mixture of (vinyl dimethicone/lauryl dimethicone) crosspolymer 20% and isododecane 80% manufactured by Shin-Etsu Chemical Co., Ltd. (Note 2) Ethyl methicone manufactured by Shin-Etsu Chemical Co., Ltd. (Note 3) Diphenylsiloxyphenyl trimethicone manufactured by Shin-Etsu Chemical Co., Ltd. (Note 4) Polysilicone-22 manufactured by Shin-Etsu Chemical Co., Ltd. (Note 5) (Methyl/phenyl) polysilsesquioxane manufactured by Shin-Etsu Chemical Co., Ltd. | | |

### (Production Method)

The components 1 to 11 were uniformly mixed to prepare a sunscreen gel.

The sunscreen gel was a cosmetic with an emollient feel, high usability, and a good soft focus effect.

### [Example 18] Stick Concealer

| | Composition | % |
|---|---|---|
| 1. | Production Example 7 | 6 |
| 2. | KSP-411 (Note 1) | 10 |
| 3. | KMP-592 (Note 2) | 16 |
| 4. | Dimethicone (6 cs) | 22 |
| 5. | KF-4418 (Note 3) | 30 |
| 6. | Microcrystalline wax | 6 |
| 7. | Ceresin | 10 |
| | Total | 100.0 |

| | | |
|---|---|---|
| (Note 1) A polysilicone-1 crosspolymer manufactured by Shin-Etsu Chemical Co., Ltd. (Note 2) (Methyl/phenyl) polysilsesquioxane manufactured by Shin-Etsu Chemical Co., Ltd. (Note 3) Caprylyl methicone manufactured by Shin-Etsu Chemical Co., Ltd. | | |

### (Production Method)

The components 1 to 7 were uniformly mixed at 95°C and were charged into a stick container to prepare a stick concealer.

The stick concealer was a cosmetic with an emollient feel, high usability, and a good soft focus effect.

### [Example 19] Lipstick

| | Composition | % |
|---|---|---|
| 1. | Production Example 1 | 5 |
| 2. | Polyethylene | 7 |
| 3. | Microcrystalline wax | 3 |
| 4. | KP-561P (Note 1) | 10 |
| 5. | Diisostearyl malate | 13 |
| 6. | Neopentyl glycol diethylhexanoate | 15 |
| 7. | Neopentyl glycol dicaprate | 7 |
| 8. | Hydrogenated polyisobutene | Balance |
| 9. | KF-54HV (Note 2) | 8 |
| 10. | Pearl pigment | 5 |
| 11. | Mica | 0.3 |
| 12. | Red 202 | 0.1 |
| 13. | Yellow 4 | 0.3 |
| 14. | KP-574 (Note 3) treated titanium oxide | 0.8 |
| 15. | KP-574 (Note 3) treated black iron oxide | 0.06 |
| 16. | Red 201 | 0.04 |
| 17. | KP-574 (Note 3) treated red oxide | 0.2 |
| 18. | Triisostearate polyglyceryl-2 | 1.2 |
| | Total | 100.0 |

| | | |
|---|---|---|
| (Note 1) An (acrylates/stearyl acrylate/dimethicone methacrylate) copolymer manufactured by Shin-Etsu Chemical Co., Ltd. (Note 2) Diphenyl dimethicone manufactured by Shin-Etsu Chemical Co., Ltd. (Note 3) An (acrylates/tridecyl acrylate/triethoxysilylpropyl methacrylate/dimethicone methacrylate) copolymer manufactured by Shin-Etsu Chemical Co., Ltd. | | |

### (Production Method)

A: The components 11 to 18 were dispersed using a roll.
B: The components 1 to 9 were uniformly mixed at 95°C.
C: The mixture prepared in A and 10 were added to the mixture prepared in B and were cooled to prepare a lipstick.

The lipstick was a cosmetic with an emollient feel, high usability, and a good soft focus effect.

### [Example 20] Mascara

| | Composition | % |
|---|---|---|
| 1. | Production Example 2 | 5 |
| 2. | TSPL-30-ID (Note 1) | 10 |
| 3. | Isododecane | Balance |
| 4. | Dextrin palmitate | 2 |
| 5. | Ceresin | 7 |
| 6. | Microcrystalline wax | 5 |
| 7. | Disteardimonium hectorite | 5 |
| 8. | Alkylsilane-treated black iron oxide (Note 2) | 5 |
| 9. | Alkylsilane-treated talc (Note 2) | 5 |
| 10. | KMP-590 (Note 3) | 5 |
| 11. | KF-6017 (Note 4) | 1.2 |
| 12. | Propylene carbonate | 1.6 |
| 13. | Phenoxvethanol | 0.2 |
| | Total | 100.0 |

| | | |
|---|---|---|
| (Note 1) Tri(trimethylsiloxy)silylpropylcarbamic acid pullulan manufactured by Shin-Etsu Chemical Co., Ltd. (label name) (Note 2) manufactured by Shin-Etsu Chemical Co., Ltd., treated with AES-3083 (Note 3) Polymethylsilsesquioxane manufactured by Shin-Etsu Chemical Co., Ltd. (Note 4) PEG-10 dimethicone manufactured by Shin-Etsu Chemical Co., Ltd. | | |

### (Production Method)

A: The components 1 to 7 were uniformly mixed at 90°C.
B: The components 8 to 13 were added to the mixture prepared in A and were uniformly mixed to prepare a mascara.

The mascara had high usability, applicability, and storage stability, and was a cosmetic with an emollient feel, high usability, and a good soft focus effect.

### [Example 21] Powder Foundation

| | Composition | % |
|---|---|---|
| 1. | Ethylhexyl methoxycinnamate | 4 |
| 2. | KF-56A (Note 1) | 4.5 |
| 3. | Triethylhexanoin | 1.5 |
| 4. | KP-561P (Note 2) | 1 |
| 5. | Production Example 3 | 1 |
| 6. | Silicone-treated mica (Note 3) | 30 |
| 7. | Ba sulfate | 10 |
| 8. | KSP-300 (Note 4) | 5 |
| 9. | KSP-100 (Note 5) | 4 |
| 10. | Silicone-treated talc (Note 3) | Balance |
| 11. | Silicone-treated titanium oxide (Note 3) | 6 |
| 12. | Silicone-treated yellow iron oxide (Note 3) | 0.5 |
| 13. | Silicone-treated red iron oxide (Note 3) | 0.2 |
| 14. | Silicone-treated black iron oxide (Note 3) | 0.1 |
| | Total | 100.0 |

| | | |
|---|---|---|
| (Note 1) Diphenylsiloxyphenyl trimethicone manufactured by Shin-Etsu Chemical Co., Ltd. (Note 2) An (acrylates/stearyl acrylate/dimethicone methacrylate) copolymer manufactured by Shin-Etsu Chemical Co., Ltd. (Note 3) manufactured by Shin-Etsu Chemical Co., Ltd., treated with KF-9909 (Note 4) A (diphenyl dimethicone/vinyl diphenyl dimethicone/silsesquioxane) crosspolymer manufactured by Shin-Etsu Chemical Co., Ltd. (Note 5) A (vinyl dimethicone/methicone silsesquioxane) crosspolymer manufactured by Shin-Etsu Chemical Co., Ltd. | | |

### (Production Method)

A: The components 1 to 5 were heated to 50°C, were uniformly mixed, and were cooled to room temperature.
B: The components 6 to 14 were uniformly mixed.
C: The mixture prepared in A was added to the mixture prepared in B and was uniformly mixed in a Henschel mixer. The resulting powder was passed through a mesh and was then molded into a metal plate using a mold to prepare a powder foundation.

The powder foundation was a cosmetic with an emollient feel, high usability, and a good soft focus effect.

### [Example 22] Hair Oil

| | Composition | % |
|---|---|---|
| 1. | Production Example 4 | 3 |
| 2. | KF-56A (Note 1) | 7 |
| 3. | Diethylhexyl succinate | 10 |
| 4. | X-21-5613 (Note 2) | 1.5 |
| 5. | Tocopherol | 0.1 |
| 6. | Perfume | 0.1 |
| 7. | Light liquid isoparaffin | Balance |
| | Total | 100.0 |

| | | |
|---|---|---|
| (Note 1) Diphenylsiloxyphenyl trimethicone manufactured by Shin-Etsu Chemical Co., Ltd. (Note 2) Dimethiconol manufactured by Shin-Etsu Chemical Co., Ltd. | | |

### (Production Method)

The components 1 to 7 were uniformly mixed to prepare a hair oil.

The hair oil was a cosmetic with an emollient feel, high usability, and a good soft focus effect.

### [Example 23] Hair Treatment

| | Composition | % |
|---|---|---|
| 1. | Production Example 5 | 1 |
| 2. | Cetanol | 2 |
| 3. | Cetyl octanoate | 3 |
| 4. | Butyl p-hydroxybenzoate | 0.1 |
| 5. | KF-56A (Note 1) | 1 |
| 6. | Behentrimonium chloride | 1 |
| 7. | Propylene glycol | 5 |
| 8. | Hydroxyethyl cellulose | 0.1 |
| 9. | Water | Balance |
| 10. | X-52-2328 (Note 2) | 4 |
| 11. | Perfume | q.s. |
| | Total | 100.0 |

| | | |
|---|---|---|
| (Note 1) Diphenylsiloxyphenyl trimethicone manufactured by Shin-Etsu Chemical Co., Ltd. (Note 2) Aminopropyl dimethicone manufactured by Shin-Etsu Chemical Co., Ltd. | | |

### (Production Method)

A: The components 6 to 9 were heated to 70°C and were uniformly mixed.
B: The components 1 to 5 were heated to 70°C and were uniformly mixed.
C: The mixture prepared in B was added to the mixture prepared in A, was emulsified, and was gradually cooled, and the components 10 and 11 were then added thereto to prepare a hair treatment.

The hair treatment was a cosmetic with an emollient feel, high usability, and a good soft focus effect.

### [Example 24] W/O Cream

| | Composition | % |
|---|---|---|
| 1. | KSG-710 (Note 1) | 6 |
| 2. | Production Example 2 | 2 |
| 3. | Octyldodecyl myristate | 13.5 |
| 4. | Macadamia nut oil | 5 |
| 5. | KF-6038 (Note 2) | 1 |
| 6. | KSP-100 (Note 3) | 2 |
| 7. | KMP-590 (Note 4) | 1 |
| 8. | Sodium citrate | 0.2 |
| 9. | DPG | 8 |
| 10. | Diglycerol | 3 |
| 11. | Water | 58.3 |
| | Total | 100.0 |

| | | |
|---|---|---|
| (Note 1) A mixture of dimethicone (6 cs, kinematic viscosity at 25°C: 6 mm²/s) 70% to 80% + (dimethicone/polyglycerol-3) crosspolymer 20% to 30% manufactured by Shin-Etsu Chemical Co., Ltd. (Note 2) Lauryl PEG-9 polydimethylsiloxyethyl dimethicone manufactured by Shin-Etsu Chemical Co., Ltd. (Note 3) A (vinyl dimethicone/methicone silsesquioxane) crosspolymer manufactured by Shin-Etsu Chemical Co., Ltd. (Note 4) Polymethylsilsesquioxane manufactured by Shin-Etsu Chemical Co., Ltd. | | |

### (Production Method)

A: The components 1 to 7 were uniformly mixed.
B: The components 8 to 11 were uniformly mixed.
C: While stirring, the mixture prepared in B was gradually added to the mixture prepared in A and was emulsified.

The resulting W/O cream was a cosmetic with an emollient feel, high usability, and a good soft focus effect.

### [Example 25] W/O Cream Foundation

| | Composition | % |
|---|---|---|
| 1. | KSG-310 (Note 1) | 2 |
| 2. | Production Example 5 | 2 |
| 3. | Mineral oil | 2 |
| 4. | Triethylhexanoin | 5 |
| 5. | Isotridecyl isononanoate | 9 |
| 6. | KF-6038 (Note 2) | 1.5 |
| 7. | KMP-591 (Note 3) | 1 |
| 8. | KSP-100 (Note 4) | 2 |
| 9. | KTP-09W, R, Y, B (Note 5) | 10 |
| 10. | BG | 5 |
| 11. | Sodium citrate | 3 |
| 12. | Magnesium sulfate | 3 |
| 13. | Water | 54.5 |
| | Total | 100.0 |

| | | |
|---|---|---|
| (Note 1) A mixture of mineral oil 65% to 75% + (PEG-15/lauryl dimethicone) crosspolymer 25% to 35% manufactured by Shin-Etsu Chemical Co., Ltd. (Note 2) Lauryl PEG-9 polydimethylsiloxyethyl dimethicone manufactured by Shin-Etsu Chemical Co., Ltd. (Note 3) Polymethylsilsesquioxane manufactured by Shin-Etsu Chemical Co., Ltd. (Note 4) A (vinyl dimethicone/methicone silsesquioxane) crosspolymer manufactured by Shin-Etsu Chemical Co., Ltd. (Note 5) KF-9909-treated color inorganic pigments W: white, R: red, Y: yellow, B: black each manufactured by Shin-Etsu Chemical Co., Ltd. | | |

### (Production Method)

A: The components 1 to 6 were uniformly mixed, and the components 7 to 9 were added thereto and were homogenized.
B: The components 10 to 13 were uniformly mixed.
C: While stirring, the mixture prepared in B was gradually added to the mixture prepared in A and was emulsified.

The resulting W/O cream foundation was a cosmetic with an emollient feel, high usability, and a good soft focus effect.

### [Example 26] W/O Water Break Cream

| | Composition | % |
|---|---|---|
| 1. | KSG-840 (Note 1) | 3 |
| 2. | KF-6048 (Note 2) | 0.2 |
| 3. | Production Example 6 | 1 |
| 4. | Squalane | 10.8 |
| 5. | BG | 8 |
| 6. | Ethanol | 5 |
| 7. | Sodium citrate | 0.2 |
| 8. | Sodium chloride | 0.5 |
| 9. | Water | 71.3 |
| | Total | 100.0 |

| | | |
|---|---|---|
| (Note 1) A mixture of squalane 65% to 75% + (lauryl dimethicone/polyglycerol-3) crosspolymer 25% to 35% manufactured by Shin-Etsu Chemical Co., Ltd. (Note 2) Cetyl PEG/PPG-10/1 dimethicone manufactured by Shin-Etsu Chemical Co., Ltd. | | |

### (Production Method)

A: The components 1 to 4 were uniformly mixed.
B: The components 5 to 9 were uniformly mixed.
C: The mixture prepared in B was added to the mixture prepared in A and was emulsified to prepare a W/O water break cream.

The W/O water break cream was a cosmetic with an emollient feel, high usability, and a good soft focus effect.

### [Example 27] W/O Cream Foundation

| | Composition | % |
|---|---|---|
| 1. | KSG-270 (Note 1) | 3.5 |
| 2. | KF-6028 (Note 2) | 3 |
| 3. | Production Example 7 | 6 |
| 4. | Organic modified clay mineral | 1.2 |
| 5. | Cyclopentasiloxane | 20 |
| 6. | Ethylhexyl methoxycinnamate | 7.5 |
| 7. | KF-7312J (Note 3) | 2 |
| 8. | KMP-591 (Note 4) | 2 |
| 9. | Ethylhexyl palmitate | 7 |
| 10. | KP-578 (Note 5) | 0.2 |
| 11. | KTP-09W, R, Y, B (Note 6) | 10 |
| 12. | BG | 5 |
| 13. | Methylparaben | 0.15 |
| 14. | Sodium citrate | 0.2 |
| 15. | Sodium chloride | 0.5 |
| 16. | Water | 54.5 |
| | Total | 100.0 |

| | | |
|---|---|---|
| (Note 1) A mixture of diphenylsiloxyphenyl trimethicone 75-85% + (dimethicone/(PEG-10/15)) crosspolymer 15% to 25% manufactured by Shin-Etsu Chemical Co., Ltd. (Note 2) PEG-9 polydimethylsiloxyethyl dimethicone manufactured by Shin-Etsu Chemical Co., Ltd. (Note 3) A solution of 50% trimethylsiloxysilicic acid in cyclopentasiloxane manufactured by Shin-Etsu Chemical Co., Ltd. (Note 4) Polymethylsilsesquioxane manufactured by Shin-Etsu Chemical Co., Ltd. (Note 5) An (acrylates/ethylhexyl acrylate/dimethicone methacrylate) copolymer manufactured by Shin-Etsu Chemical Co., Ltd. (Note 6) KF-9909-treated color inorganic pigments W: white, R: red, Y: yellow, B: black each manufactured by Shin-Etsu Chemical Co., Ltd. | | |

### (Production Method)

A: The components 9 to 11 were dispersed with a rolling mill.
B: The components 1 to 8 were uniformly mixed.
C: The components 12 to 16 were uniformly mixed.
D: The mixture prepared in C was added to the mixture prepared in B and was emulsified, and A was added thereto to prepare a W/O cream foundation.

The W/O cream foundation was a cosmetic with an emollient feel, high usability, and a good soft focus effect.

### [Example 28] O/W Foundation Cream

| | Composition | % |
|---|---|---|
| 1. | Production Example 2 | 0.3 |
| 2. | KMP-592 (Note 1) | 10 |
| 3. | Polysorbate-60 | 2 |
| 4. | A sodium acrylate-sodium acryloyldimethyltaurate copolymer composition (Note 2) | 1 |
| 5. | 5% aqueous solution of (acryloyldimethyltaurine ammonium/VP) copolymer (Note 3) | 15 |
| 6. | Butylene glycol | 10 |
| 7. | Glycerol | 3 |
| 8. | Pentylene glycol | 1 |
| 9. | Methylparaben | 0.15 |
| 10. | Purified water | 57.55 |
| | Total | 100.0 |

| | | |
|---|---|---|
| (Note 1) (Methyl/phenyl) polysilsesquioxane manufactured by Shin-Etsu Chemical Co., Ltd. (Note 2) SIMULGEL EG manufactured by SEPPIC (Note 3) 5% aqueous solution of Aristoflex AVC manufactured by Clariant | | |

### (Production Method)

A: The components 3 to 10 were uniformly mixed.
B: The components 1 and 2 were uniformly mixed.
C: The mixture prepared in B was added to the mixture prepared in A and was emulsified to prepare an O/W foundation cream.

The O/W foundation cream was a cosmetic with an emollient feel, high usability, and a good soft focus effect.

### [Example 29] O/W Cream

| | | |
|---|---|---|
| | Composition | % |
| 1. | Production Example 1 | 25 |
| 2. | Production Example 5 | 15 |
| 3. | Isotridecyl isononanoate | 10 |
| 4. | BG | 7 |
| 5. | KF-6100 (Note 1) | 0.7 |
| 6. | PEG-60 hydrogenated castor oil | 0.5 |
| 7. | A sodium acrylate-sodium acryloyldimethyltaurate copolymer composition (Note 2) | 0.8 |
| 8. | 5% aqueous solution of (acryloyldimethyltaurine ammonium/VP) copolymer (Note 3) | 10 |
| 9. | PCA-Na aqueous solution | 0.5 |
| 10. | Ethylhexyl glycerol | 0.1 |
| 11. | Purified water | Balance |
| | Total | 100.0 |

| | | |
|---|---|---|
| (Note 1) Polyglyceryl-3 disiloxane dimethicone manufactured by Shin-Etsu Chemical Co., Ltd. (Note 2) SIMULGEL EG manufactured by SEPPIC (Note 3) 5% aqueous solution of Aristoflex AVC manufactured by Clariant | | |

### (Production Method)

A: The components 3 to 10 were uniformly mixed.
B: The components 1 to 2 were uniformly mixed.
C: The mixture prepared in B was added to the mixture prepared in A and was uniformly mixed.

The O/W cream was a cosmetic with an emollient feel, high usability, and a good soft focus effect.

### [Example 30] W/O Concealer

| | Composition | % |
|---|---|---|
| 1. | KSG-710 (Note 1) | 6 |
| 2. | Production Example 1 | 25 |
| 3. | KSG-19 (Note 2) | 20 |
| 4. | KF-6104 (Note 3) | 1 |
| 5. | TMF-1.5 (Note 4) | Balance |
| 6. | Hexyl laurate | 5 |
| 7. | Mg sulfate | 0.4 |
| 8. | Na citrate | 0.1 |
| 9. | Glycerol | 2 |
| 10. | Pentylene glycol | 2 |
| 11. | Water | 25.5 |
| | Total | 100.0 |

| | | |
|---|---|---|
| (Note 1) A mixture of dimethicone (6 cs) 70% to 80% + (dimethicone/polyglycerol-3) crosspolymer 20% to 30% manufactured by Shin-Etsu Chemical Co., Ltd. (Note 2) A (dimethicone/vinyl dimethicone) crosspolymer mixture manufactured by Shin-Etsu Chemical Co., Ltd. (Note 3) Polyglyceryl-3 polydimethylsiloxyethyl dimethicone manufactured by Shin-Etsu Chemical Co., Ltd. (Note 4) Methyl trimethicone manufactured by Shin-Etsu Chemical Co., Ltd. | | |

### (Production Method)

A: The components 1 to 6 were uniformly mixed.
B: The components 7 to 11 were uniformly mixed.
C: While stirring, the mixture prepared in B was gradually added to the mixture prepared in A and was emulsified.

The resulting W/O concealer was a cosmetic with an emollient feel, high usability, and a good soft focus effect.

## Claims

1. A cosmetic comprising a silicone gel in which the following component (a) is swollen with the following component (b):
(a) a cross-linked silicone in which a silicone chain having an alkyl branch with 8 to 30 carbon atoms is cross-linked with a silicone chain: 5% to 80% by mass in the gel, and
(b) one or more oil agents selected from the following (b1) and (b2): 20% to 95% by mass in the gel,
(b1) a hydrocarbon oil with a boiling point in the range of 190°C to 260°C and a kinematic viscosity in the range of 1.5 to 10 mm²/s at 25°C, and
(b2) an ester oil with an IOB value in the range of 0.05 to 0.3 and a kinematic viscosity in the range of 1.5 to 25 mm²/s at 25°C.

2. The cosmetic according to claim 1, wherein the component (a) is one or more selected from a (vinyl dimethicone/lauryl dimethicone) crosspolymer and a (lauryl polydimethylsiloxyethyl dimethicone/bisvinyl dimethicone) crosspolymer.

3. The cosmetic according to claim 1, wherein the component (b) is one or more selected from undecane, tridecane, a C13-15 alkane, a cocoalkyl (caprylate/caprate), and (caprylic/capric) triglyceride.

4. The cosmetic according to claim 1, wherein a total amount of the component (a) and the component (b) in the silicone gel ranges from 80% to 100% by mass.

5. The cosmetic according to claim 1, wherein the silicone gel has a total light transmittance in the range of 80% to 99% at a thickness of 500 µm as measured by a method described in JIS K 7361-1: 1997 and a haze in the range of 40% to 95% as measured by a method described in JIS K 7136: 2000.

6. The cosmetic according to any one of claims 1 to 5, wherein the cosmetic is a non-aqueous cosmetic.

7. The cosmetic according to any one of claims 1 to 5, which is a water-in-oil cosmetic, comprising:
the silicone gel: 0.1% to 50% by mass;
(c) an aqueous component: 30% to 90% by mass; and
(d) a silicone surfactant other than the component (a): 0.1% to 20% by mass.

8. The cosmetic according to any one of claims 1 to 5, which is an oil-in-water cosmetic, comprising:
the silicone gel: 0.1% to 50% by mass;
(c) an aqueous component: 40% to 96% by mass; and
(e) a water-soluble thickener: 0.01% to 10% by mass.
